# FASCICULE DE BREVET EUROPEEN

(11) **EP 2 699 221 B1**
(45) Date de publication et mention de la délivrance du brevet: **08.02.2017**
(21) Numéro de dépôt: 12722443.4
(22) Date de dépôt: 20.04.2012
(51) Int. Cl.: A61K 8/60, A61Q 19/00, A61Q 19/08, C12Q 1/68

(54) **SIGNATURE MOLÉCULAIRE DES TACHES PIGMENTAIRES CUTANÉES, ASSOCIÉE À LA MATRICE EXTRACELLULAIRE**
MOLEKULARE SIGNATUR FÜR HAUTPIGMENTIERUNG IN KOMBINATION MIT DER EXTRAZELLULÄREN MATRIX
MOLECULAR SIGNATURE FOR SKIN PIGMENT SPOTS, WHICH IS COMBINED WITH THE EXTRACELLULAR MATRIX

(30) Priorité: 22.04.2011 FR 1153533; 22.04.2011 FR 1153537; 08.06.2011 US 201161494441 P; 08.06.2011 US 201161494438 P
(43) Date de publication de la demande: 26.02.2014
(73) Titulaire: L'Oréal, 75008 Paris (FR)
(72) Inventeur: BERNERD, Françoise, 06100 Nice (FR); DUVAL, Christine, 95170 Deuil-La-Barre (FR); DE LACHARRIERE, Olivier, 75015 Paris (FR); NOUVEAU, Stéphanie, 92100 Boulogne (FR); MARAT, Xavier, 75010 Paris (FR)
(74) Mandataire: Sellin, Carole
(86) Numéro de dépôt international: PCT/FR2012/050860
(87) Numéro de publication internationale: WO 2012/172220

(56) Documents cités:
- WO-A1-2011/026909
- WO-A2-03/089601
- WO-A2-2009/140550
- US-A1- 2008 274 908
- LIN SHI-LUNG ET AL: "CHAPTER 4: Recent Application of Intronic MicroRNA Agents in Cosmetics", 1 janvier 2008 (2008-01-01), CURRENT PERSPECTIVES IN MICRORNAS (MIRNA), SPRINGER NETHERLANDS, NL, PAGE(S) 51 - 72, XP009136584, ISBN: 978-1-4020-8532-1 abrégé page 63 - page 69
- ZHOU SHUANHU ET AL: "Comparison of TGF-beta/BMP pathways signaled by demineralized bone powder and BMP-2 in human dermal fibroblasts", JOURNAL OF BONE AND MINERAL RESEARCH, AMERICAN SOCIETY FOR BONE AND MINERAL RESEARCH, NEW YORK, NY, US, vol. 19, no. 10, 1 octobre 2004 (2004-10-01), pages 1732-1741, XP002516155, ISSN: 0884-0431, DOI: 10.1359/JBMR.040702 [extrait le 2004-07-07]
- REICHLE A ET AL: "Pioglitazone and rofecoxib combined with angiostatically scheduled trofosfamide in the treatment of far-advanced melanoma and soft tissue sarcoma", CANCER, AMERICAN CANCER SOCIETY, PHILADELPHIA, PA, US, vol. 101, no. 10, 15 novembre 2004 (2004-11-15), pages 2247-2256, XP002438977, ISSN: 0008-543X, DOI: 10.1002/CNCR.20574
- WU X N ET AL: "Experimental studies of oxymatrine and its mechanisms of action in hepatitis B and C viral infections", CHINESE JOURNAL OF DIGESTIVE DISEASES, BLACKWELL SCIENCE ASIA, CARLTON SOUTH, AU, vol. 5, no. 1, 1 février 2004 (2004-02-01) , pages 12-16, XP002551413, ISSN: 1443-9611, DOI: 10.1111/J.1443-9573.2004.00146.X [extrait le 2004-02-10]
- FAIOLA BRENDA ET AL: "PPAR alpha, more than PPAR delta, mediates the hepatic and skeletal muscle alterations induced by the PPAR agonist GW0742", TOXICOLOGICAL SCIENCES, ACADEMIC PRESS, SAN DIEGO, FL, US, vol. 105, no. 2, 1 octobre 2008 (2008-10-01), pages 384-394, XP002602234, ISSN: 1096-6080, DOI: 10.1093/TOXSCI/KFN130 [extrait le 2008-06-30]
- CHEN Y W ET AL: "Tetrandrine inhibits activation of rat hepatic stellate cells stimulated by transforming growth factor-beta in vitro via up-regulation of Smad 7", JOURNAL OF ETHNOPHARMACOLOGY, ELSEVIER SCIENTIFIC PUBLISHERS LTD, IE, vol. 100, no. 3, 14 septembre 2005 (2005-09-14), pages 299-305, XP025269903, ISSN: 0378-8741, DOI: 10.1016/J.JEP.2005.03.027 [extrait le 2005-09-14]
- JUNG ET AL: "Wen-Pi-Tang-Hab-Wu-Ling-San extract inhibits the release of inflammatory mediators from LPS-stimulated mouse macrophages", JOURNAL OF ETHNOPHARMACOLOGY, ELSEVIER SCIENTIFIC PUBLISHERS LTD, IE, vol. 114, no. 3, 2 novembre 2007 (2007-11-02), pages 439-445, XP022326516, ISSN: 0378-8741
- HASEGAWA T ET AL: "SB-431542 inhibits TGF-beta-induced contraction of collagen gel by normal and keloid fibroblasts", JOURNAL OF DERMATOLOGICAL SCIENCE, ELSEVIER SCIENCE PUBLISHERS, SHANNON, IE, vol. 39, no. 1, 1 juillet 2005 (2005-07-01), pages 33-38, XP025305650, ISSN: 0923-1811, DOI: 10.1016/J.JDERMSCI.2005.01.013 [extrait le 2005-07-01]
- LAY ING-SHIOW ET AL: "Salvianolic acid B enhances in vitro angiogenesis and improves skin flap survival in Sprague-Dawley rats.", JOURNAL OF SURGICAL RESEARCH, vol. 115, no. 2, décembre 2003 (2003-12), pages 279-285, XP002662848, ISSN: 0022-4804
- Bakala Joanna.: "Cosmetic use of a natural tetrapeptide to prevent skin aging and to improve skin", , 22 novembre 2008 (2008-11-22), XP002662849, Extrait de l'Internet: URL:http://web.archive.org/web/20081122151 619/http://www.fist.fr/fr/health-care-cosm etics/cosmetic-use-of-a-natural-tetrapepti de-to-prevent-skin-aging-and-to-improve-sk in.html [extrait le 2011-11-04]
- J.Y. LEE ET AL: "Acquired bilateral naevus of Ota-like macules: an immunohistological analysis of dermal melanogenic paracrine cytokine networks", BRITISH JOURNAL OF DERMATOLOGY, 1 décembre 2010 (2010-12-01), pages NO-NO, XP55030480, ISSN: 0007-0963, DOI: 10.1111/j.1365-2133.2010.10066.x
- YH JANG ET AL: "Oestrogen and progesterone receptor expression in melasma: an immunohistochemical analysis", JOURNAL OF THE EUROPEAN ACADEMY OF DERMATOLOGY AND VENEREOLOGY, vol. 24, no. 11, 1 novembre 2010 (2010-11-01), pages 1312-1316, XP55030479, ISSN: 0926-9959, DOI: 10.1111/j.1468-3083.2010.03638.x
- SANTOIANNI PIETRO ET AL: "Intradermal drug delivery by low-frequency sonophoresis (25 kHz).", DERMATOLOGY ONLINE JOURNAL 2004, vol. 10, no. 2, 2004, page 24, ISSN: 1087-2108

## Description

La présente invention est dans le domaine cosmétique, elle est relative à la peau. Elle s'inscrit plus généralement dans le cadre de la caractérisation des taches pigmentaires de la peau et du traitement de ces taches.

La couleur de la peau est principalement due à la présence d'un pigment, la mélanine dans l'épiderme. La mélanine est synthétisée par des cellules dendritiques spécifiques localisées dans la couche basale de l'épiderme, les mélanocytes. La mélanogénèse prend place dans des organelles, les mélanosomes qui, chargés de mélanine, sont transférés aux cellules voisines épidermiques, les kératinocytes, via les dendrites. La couleur de la peau, ou pigmentation constitutive varie selon les individus en fonction de la quantité de mélanine produite ainsi que de la nature chimiques de mélanines. Les mélanines sont des macromolécules formées à partir de tyrosine (eumélanine) ou de tyrosine et de cystéine (pheomélanine). Les mécanismes de synthèse mettent en jeu des enzymes dont les principales sont la tyrosinase et la protéine associée à la tyrosinase (Tyrp-1). Naturellement, la pigmentation de la peau est stimulée par l'exposition au soleil, c'est le phénomène de bronzage.

Il existe cependant des situations où le processus de pigmentation est altéré, et qui peuvent aboutir à des défauts de pigmentation, hypopigmentations (vitiligo, albinisme) ou à l'inverse à un excès de pigmentation, hyperpigmentations. Parmi les désordres hyperpigmentaires bénins, caractérisés par une accumulation anormale (hors bronzage) de mélanine, on peut citer le lentigo actinique, le mélasma, les séquelles pigmentaires d'acné, la pigmentation post-inflammatoires, la dermite des prés, la pigmentation liée à la plante poison ivy ou encore les dyschromies bénignes du visage.

Le lentigo actinique, également dénommé lentigo sénile ou solaire, tache sénile, tache de vieillesse, ou encore communément dénommé "crasse sénile", "marguerites de cimetière" ou "fleurs de cimetière", est de loin la plus fréquente des lésions pigmentaires. Ce type de lésions apparaît sur des zones de la peau qui ont été photoexposées, telles que le visage, le dos des mains, les membres supérieurs et notamment la face dorsale des avant-bras, le dos, et en particulier le haut du dos. Elles touchent en général les individus à partir de 40 ans. Macroscopiquement, les lentigos actiniques sont représentés par des macules pigmentées bénignes de couleur brune plus ou moins foncée, dont les bords sont nets mais irréguliers. De taille très variable, ils peuvent s'étendre de quelques millimètres à plus de deux centimètres. Malgré sa grande fréquence, peu d'études ont été publiées sur la physiologie et la pathogenèse des lentigos actiniques. Sur la base des rares études histologiques existantes, il est connu qu'il existe une augmentation de la charge mélanique épidermique globale et plus particulièrement au niveau de la couche basale. Il existe également un allongement des crêtes épidermiques qui peuvent prendre un aspect en massue dans le derme papillaire *[Montagna 1980, ber Rahman* 1996, *Andersen* 1997, *Cario-Andre 2004].* Enfin, il peut y avoir des anastomoses entre des crêtes adjacentes donnant un aspect en pont ou en réseau.

Une incontinence pigmentaire avec présence de mélanine et de mélanophages peut également exister dans le derme.

### Les traitements actuels :

Les troubles pigmentaires cutanés bénins sont généralement considérés comme inesthétiques.

Du fait de l'association avérée entre l'apparition des lentigos actiniques et l'exposition chronique au soleil, la prévention de leur apparition passe en général par l'application topique de produits dits photoprotecteurs comme les écrans solaires. Dans le cas de traitements « curatifs », de nombreux procédés, principalement à visées cosmétiques, ont donc été développés afin de tenter de les éliminer ou de réduire leur présence. L'élimination ou la réduction de la présence de ces troubles repose, d'ordinaire, sur l'application de traitements dépigmentants, basés sur la réduction de l'activité de synthèse de la mélanine dans les mélanocytes. Les molécules dépigmentantes interfèrent avec une ou des étapes de la mélanogénèse. L'une des voies des principaux produits utilisés à ce jour repose sur l'inhibition de la tyrosinase, une des enzymes clés du processus de mélanogénèse. Le but de ces traitements est de diminuer voire de stopper la synthèse de pigment.

Les principales substances dépigmentantes connues sont l'hydroquinone et ses dérivés, l'acide kojique, l'arbutine, les iminophénoles, l'acide ascorbique et ses dérivés, l'association de carnitine et de quinone, les dérivés d'amino-phénol, et les dérivés de benzothiazole, des extraits naturels, des corticoïdes, .... On trouve souvent aussi associés à ces actifs des exfoliants permettant d'augmenter la desquamation, et ainsi d'éliminer plus facilement la mélanine présente dans le stratum cornéum.

Une autre méthode de traitement non cosmétique consiste à détruire les lésions par des moyens physiques ou chimiques en utilisant les lasers ou les peelings. Cependant, ce sont des procédures assez lourdes qui ne s'attaquent pas à l'étiologie du désordre. Dans la majorité des cas, les lentigos actiniques réapparaissent quelques temps après le traitement.

Par ailleurs, il existe des inconvénients majeurs aux traitements existants. Les substances dépigmentantes peuvent présenter notamment une certaine instabilité, une faible efficacité à faible concentration, une activité biologique touchant d'autres fonctions, des propriétés toxiques ou allergisantes.

De plus, du fait qu'ils ne ciblent pas la cause profonde ayant entrainé le dérèglement de la pigmentation, il existe une grande hétérogénéité de réponse aux différents traitements dépigmentants. Ainsi, pour un traitement donné, administré par exemple de façon topique, un trouble pigmentaire cutané bénin donné, comme un lentigo actinique ou un mélasma, peut s'atténuer ou disparaître, alors qu'un autre n'évoluera pas. Cette variabilité peut être observée entre des lésions chez des individus différents mais aussi chez un même individu, entre différentes lésions. Il existe donc des troubles pigmentaires cutanés bénins pour lesquels aucun traitement topique n'est efficace à ce jour.

Il existe donc un besoin de trouver des traitements plus efficaces et plus inoffensifs pour ce type de lésion.

Pour cela, il est nécessaire d'identifier les dérégulations moléculaires et dysfonctionnements fonctionnels pouvant exister dans ce type de désordre pigmentaire afin de pouvoir identifier de nouvelles cibles et de sélectionner des actifs corrigeant ces défauts.

Dans l'art antérieur, concernant le traitement des taches pigmentaires et plus particulièrement des lentigos actiniques, il est décrit la stimulation au niveau génomique ou protéinique de molécules qui sont étroitement associées aux mélanocytes et la mélanogénèse (enzymes de la mélanogénèse, protéine du mélanosome, facteurs paracrine clé de la mélanogénèse). Elle est trouvée dans l'épiderme ou le derme pour les protéines suivantes : Tyrosinase, TRP1, DCT, Pmel-17 , POMC, ET1, ETBR, SCF, c-KIT , KGF , KGFR, Hepatocyte growth factor (HGF), MIA, TRPM1, Melan-A, Pink eye dilution, P53 et IL1α.

Par ailleurs, d'autres études décrivent des modifications moléculaires ou cellulaires dans le lentigo solaire ou sénile par des analyses de l'expression de gènes ou des protéines. Cependant, de ces différentes études, il ne ressort aucun mécanisme ou dysfonctionnement fonctionnel général sous-tendant l'apparition des taches pigmentaires.

Aucun document de l'art antérieur n'a permis de mettre en évidence de manière fiable des dérégulations moléculaires ou des dysfonctionnements fonctionnels pouvant exister dans ce type de désordre pigmentaire, au-delà des molécules étroitement associées aux mélanocytes et la mélanogénèse.

Les présents inventeurs ont mis en évidence pour la première fois l'implication de gènes dermiques liés à la matrice extracellulaire et à la jonction dermo-épidermique dans les dérèglements pigmentaires se traduisant par des taches pigmentaires de la peau, et notamment l'implication de gènes liés à la voie de signalisation TGF-beta - SMAD.

La matrice extracellulaire assure au niveau de la peau un rôle structural grâce à sa capacité à assurer le support et la cohésion des tissus et des cellules et grâce à ses propriétés mécaniques qui lui permettent de faire résistance à la traction (notamment du fait de la présence de collagènes), à la compression (notamment du fait de la présence de protéoglycanes).

Elle joue un rôle biologique important car c'est aussi un acteur majeur de la régulation de l'homéostasie épidermique et dermique. Grâce, entre autres, à ses propriétés de réservoir de facteurs de croissance et de cytokines, elle est impliquée dans la morphogénèse, la prolifération, la différentiation cellulaire et la réparation tissulaire

La jonction dermo-épidermique (JDE) quant à elle est réalisée par la membrane basale qui est constituée d'un feuillet de matrice extracellulaire et qui sépare l'épiderme du derme. Elle constitue une barrière de perméabilité et régule les échanges de molécules, en particulier de nutriments, entre les deux tissus. Elle assure une fonction d'attachement et d'ancrage de l'épiderme à la matrice sous-jacente et de cohésion structurale de l'épithélium. Elle joue aussi un rôle important dans la régulation de la différentiation, et de la migration des cellules épidermiques ainsi que les étapes de morphogénèse de l'épiderme.

Le Facteur de croissance transformant bêta (TGF-β) contrôle la prolifération, la différenciation cellulaire, et d'autres fonctions dans la plupart des cellules. Il joue un rôle dans l'immunité, le cancer.

Certaines cellules sécrètent des TGF-β, et ont aussi des récepteurs pour le TGF-β. Les Smad composent la voie de signalisation de TGF-β. Ils permettent la transduction du signal lorsque TGF-β se lie à des récepteurs membranaires. Les réponses cellulaires induites par cette voie peuvent varier pour un même type cellulaire selon le contexte cellulaire. C'est un système de signalisation très dynamique.

Dans la voie canonique, le TGF-β se lie à un récepteur membranaire de type II qui forme un complexe bidimérique avec un second récepteur de type I. Un récepteur de type III (betaglycan) aide ce processus de liaison en captant TGFbeta et en le présentant au complexe réceptoriel bidimérique. Le récepteur de type II, lié à TGF-β, phosphoryle le récepteur de type I qui propage le signal en phosphorylant à son tour des protéines cytosoliques, les Smad (Smad 2 et 3). Les Smad régulés par le récepteur de type I (R-Smad) se lient alors à un Smad commun, Smad 4 pour composer un complexe activé. Ce complexe entre alors dans le noyau cellulaire où il agit comme facteur de transcription de multiples gènes, et conduit à une réponse d'activation ou de répression.

Des récepteurs de type I spécifiques, activés par d'autres ligands tels que divers BMP (protéines morphogéniques osseuses), activent la voie de signalisation via d'autres Smad (1, 5,8).

D'une manière générale, l'activation de la voie conduit aussi à la stimulation de Smad inhibitrices tels que les Smad 6 et 7 qui assurent un rétro contrôle négatif.

Parmi les gènes cibles de la voie TGF-beta, figurent de nombreux gènes codant pour des molécules de la matrice extracellulaire, dont par exemple les collagènes, l'élastine, la fibronectine, les thrombospondines qui assurent principalement un rôle de structure, et simultanément, pour des protéines de remodelage matriciel qui participent au renouvellement et à la dégradation des molécules de la matrice.

Cette voie a aussi une action stimulatrice ou inhibitrice dans la production de nombreux facteurs de croissance et de cytokines par les cellules dermiques.

Pour la première fois, il a été montré par les présents inventeurs que certains gènes liés à la matrice extracellulaire ou à la jonction dermo-épidermique et notamment à la voie de signalisation TGF-β - SMAD, ont des niveaux de transcription différant significativement entre de la peau saine et de la peau issue d'une tache pigmentaire, mettant ainsi en évidence le lien entre dérégulation au sein de la matrice extracellulaire et de la jonction dermo-épidermique, notamment dérégulation de la signalisation TGF-β- SMAD, et modulation de la pigmentation induisant notamment l'apparition de taches pigmentaires.

On peut penser que l'augmentation ou la diminution de certaines protéines impliquées dans ces différentes voies, soit dépendantes de TGF-Beta soit des BMP, peut conduire à une signalisation accrue et/ou aberrante pouvant perturber l'homéostasie dermique, jonctionnelle et se répercuter globalement au niveau épidermique.

Sans pour autant être lié par une quelconque théorie, les modifications d'expression de différents gènes liés à cette matrice extracellulaire et à la jonction dermo-épidermique ont conduit les inventeurs à penser que, du fait du rôle joué par les protéines de la matrice et de la jonction dermo-épidermique, la dérégulation de ces gènes, signe d'une modification de tout le compartiment dermique, perturbe l'homéostasie de l'épiderme et génère des modifications importantes en particulier de l'architecture histologique de l'épiderme dans les taches pigmentaires (élongation des crêtes épidermiques dans le derme par exemple). En conséquence, la charge mélanique épidermique est augmentée et donne lieu à des taches pigmentaires.

Une signature moléculaire représentative des différences d'expression génique existant entre la peau issue de tache pigmentaire et la peau saine adjacente, et aux différentes applications et méthodes faisant usage de la connaissance de cette signature, notamment pour moduler la pigmentation de la peau, dans le traitement cosmétique des taches pigmentaires ou pour uniformiser le teint ou homogénéiser la couleur de la peau est décrite.

Cette signature est constituée des gènes suivants : TGFBR2 (récepteur II du facteur de croissance transformant béta [70/80kDa]), TGFBI (facteur de croissance transformant béta induit, 68 kDa), BMP2 (protéine morphogénique osseuse 2), SMAD3 (membre 3 de la famille SMAD), THBS2 (thrombospondine 2), TGFBR3 (récepteur III du facteur de croissance transformant béta), SEMA5A (domaine sema, 7 répétitions thrombospondine, semaphorine 5A), SMAD7 (membre 7 de la famille SMAD), SOSTDC1 (protéine 1 contenant un domaine à sclérostine), FRAS1 (Fraser syndrome 1), LEPREL1 (leprecan-like 1), MATN2 (matriline 2), DST (Dystonine, connu également sous le nom de BPAG1 PLOD2 (procollagen-lysine, 2-oxoglutarate 5-dioxygenase 2), ITGA2 (intégrine alpha 2 (CD49B, sous-unité alpha 2 du récepteur VLA-2)), COL6A3 (collagène de type 6, alpha 3), CRTAP (protéine associée au cartilage également connue sous le nom de LEPREL3), LAMC1 (laminine gamma 1, anciennement LAMB2), LAMB3 (laminine béta 3), LAMA3 (laminine alpha 3), ITGAV (intégrine alpha V (récepteur vitronectine)), ITGB1 (intégrine béta 1) et ACTN1 (actinine alpha 1). Le gène ITGB1 peut être exclu de cette liste. Le gène SEMA5A et/ou le gène ITGB1 peuvent être exclus de cette liste.

Ces gènes peuvent être regroupés fonctionnellement de la manière suivante :
- Liste A : Gènes codant pour des facteurs impliqués dans l'activation de la voie de signalisation TGF-beta - SMAD : TGFBR2, TGFBI, BMP2, SMAD3, THBS2, TGFBR3, SEMA5A, SMAD7 et SOSTDC1, le gène SEMA5A pouvant toutefois être exclu de cette liste A, et
- Liste B, regroupant les gènes FRAS1, LEPREL1, MATN2, DST, PLOD2, ITGA2, COL6A3, CRTAP, LAMC1, LAMB3, LAMA3, ITGAV, ITGB1 et ACTN1. Le gène ITGB1 est éventuellement exclu de cette liste B. Parmi la liste B, les groupements fonctionnels suivants peuvent être effectués :
   - Gènes codant pour des collagènes, composants de la matrice extracellulaire, et plus particulièrement des collagènes filamenteux du stroma et pour des molécules associées à la biosynthèse et à l'assemblage des collagènes : LEPREL1, PLOD2, COL6A3 et CRTAP ;
   - Gènes codant pour des laminines, protéines adhésives de la matrice extracellulaire: LAMC1, LAMB3 et LAMA3 ;
   - Gènes codant pour des protéines matricielles associées à la zone de la membrane basale : FRAS1, MATN2 et DST ;
   - Gènes codant pour des intégrines, impliquées dans la liaison des cellules à la matrice extracellulaire : ITGA2 et ITGAV, avec éventuellement ITGB1; et
   - Gène codant pour une actine, composant de la matrice extracellulaire : ACTN1.

Des gènes préférés au sein des gènes dermiques TGFBR2, TGFBI, BMP2, SMAD3, THBS2, TGFBR3, SEMA5A, SMAD7 et SOSTDC1, constituant la liste A, sont les gènes TGFBR2, TGFBI, BMP2, SMAD3, THBS2 et TGFBR3.

Des gènes préférés au sein des gènes dermiques FRAS1, LEPREL1, MATN2, DST, PLOD2, ITGA2, COL6A3, CRTAP, LAMC1, LAMB3, LAMA3, ITGAV, ITGB1 et ACTN1, constituant la liste B, sont les gènes FRAS1, LEPREL1, MATN2, DST, PLOD2 et ITGA2.

Lesdits gènes s'entendent des gènes humains dénommés ainsi.

Les listes de gènes A et B sont par ailleurs liées par nombre d'interactions connues entre les divers gènes constituant ces listes. Ces liens peuvent notamment être mis en évidence par un réseau d'interactions tel que généré par exemple par le logiciel IPA (Ingenuity pathway analysis) qui, à partir de données bibliographiques, permet de montrer les différents liens directs ou indirects existants entre des gènes ou des protéines. Un réseau d'interaction généré par ce logiciel IPA a en effet montré un lien, direct ou indirect, entre les gènes et/ou protéines TGFBR2, TGFBR3, SMAD3, SMAD7, BMP2, THBS2 (liste A) et MATN2, DST, ITGA2, COL6A3, LAMC1, LAMB3, ITGAV, ITGB1, ACTN1 (liste B).

Par ailleurs, différentes références bibliographiques illustrent également des interactions directes entre les gènes mentionnés dans la liste A et ceux mentionnés dans la liste B, concrétisant ainsi le lien fonctionnel unissant ces deux familles, notamment entre les gènes SMAD3 et COL6A3 (Verrecchia et al, 2001), entre les gènes TGFBR2, THBS2 et COL6A3 (Berking et al, 2001) et entre SMAD3 et LAMC1 (Kawata et al, 2002), à titre illustratif.

Les inventeurs ont en effet mis en évidence la modulation significative et reproductible du niveau d'expression des gènes des listes A et B entre de la peau issue de tache pigmentaire et de la peau saine correspondante.

Par ailleurs, la modulation des gènes de la liste A, impliqués dans la voie de signalisation TGF-β au sein de taches hyperpigmentaires tend à l'augmentation de la signalisation TGF-béta-SMAD. Les modulations observées pour les gènes de la liste A mettent donc en évidence le lien entre activation/stimulation de la voie de signalisation TGF-béta - SMAD et augmentation de la pigmentation, comme vérifié par les inventeurs dans le présent exemple 4.

Selon un premier aspect une méthode de caractérisation d'une tache pigmentaire cutanée est décrite. La présente invention concerne une méthode de caractérisation d'une tache pigmentaire de type lentigo actinique telle que définie dans la revendication 1.

Une telle méthode permet entre autres de confirmer la nature de la tache pigmentaire dans le cas où cette dernière est déjà apparente, par exemple visuellement à l'oeil nu. La méthode permet également de prédire l'apparition d'une tache, quand cette dernière n'est pas encore observée mais uniquement soupçonnée, ou bien de conclure qu'une peau est encline à la formation de taches cutanées, ou sujette à des défauts de pigmentation, par exemple quand aucune tache n'est encore apparente.

Les taches pigmentaires cutanées concernées sont des taches hyperpigmentaires, ou encore dites hyperpigmentées, correspondant à un excès de pigment, ou bien des taches hypopigmentaires, ou encore hypo-pigmentées, correspondant à des défauts de pigmentation. Des hypopigmentations sont notamment le vitiligo et l'albinisme. Des désordres hyperpigmentaires bénins caractérisés par une accumulation anormale (hors bronzage) de mélanine, sont par exemple le lentigo actinique, le mélasma, les séquelles pigmentaires d'acné, la pigmentation post-inflammatoires, la dermite des prés, la pigmentation liée à la plante poison ivy ou encore les dyschromies bénignes du visage. Les taches pigmentaires englobent également des défauts, imperfections ou irrégularités de pigmentation rendant le teint non uniforme, ou la couleur de la peau non homogène.

Les taches pigmentaires dont il est question sont préférentiellement des taches pigmentaires de la peau humaine. Toutefois, les désordres au niveau de la matrice extracellulaire et de la voie de signalisation TGF-β - SMAD dans le derme mis en évidence par les présents inventeurs étant tout-à-fait généraux, des méthodes similaires peuvent être envisagées pour d'autres espèces animales touchées également par les taches pigmentaires. Dans ce cas, les différentes méthodes, utilisations ou compositions seront mises en oeuvre avec les gènes de l'espèce considérée, orthologues aux gènes humains mentionnés ci-dessus.

La méthode comprend la comparaison des niveaux d'expression au sein de la peau issue de ladite tache et au sein de la peau non lésée, de préférence adjacente, du même individu, d'au moins un gène dermique lié à la matrice extracellulaire choisi parmi la liste constituée des gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1. Alternativement, le gène dermique peut être choisi parmi la liste A constituée de gènes liés à la voie de signalisation TGF-β - SMAD; ou bien parmi la liste B constituée des gènes FRAS1, LEPREL1, MATN2, DST, PLOD2, ITGA2, COL6A3, CRTAP, LAMC1, LAMB3, LAMA3, ITGAV et ACTN1. Selon certaines mises en oeuvre, SEMA5A est exclu de ces listes. Il peut alternativement ou en sus en être de même pour le gène ITGB1. Les gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1 partagent un rôle dans la structure ITGAV, ITGB1 et ACTN1 partagent un rôle dans la structure ou le renouvellement de la matrice extracellulaire, notamment il s'agit de gènes impliqués dans l'activation de la voie de signalisation TGF-β - SMAD, ou de gènes codant pour des composants de la matrice extracellulaire ou des gènes codant pour des protéines impliquées dans la synthèse des composants de cette matrice, ou bien liées au renouvellement de cette matrice conjonctive ou encore de gènes codant pour des protéines matricielles associées à la jonction dermo-épidermique et à la zone de la membrane basale.

Par ailleurs, lesdits gènes sont dits gènes dermiques du fait qu'il s'agit de gènes exprimés majoritairement dans le derme et donnant lieu à des protéines caractéristiques du compartiment dermique ou bien de la jonction dermo-épidermique quant à sa face dermique ; et ce par opposition par exemple à des protéines kératinocytaires, à des protéines intracellulaires ou à des protéines épidermiques notamment, telles que des protéines exprimées préférentiellement dans le stratum corneum.

Les niveaux d'expression de l'un au moins des gènes sont mesurés au niveau de la peau issue de tache avérée ou supposée, et au niveau de la peau adjacente non lésée. De préférence, les niveaux sont mesurés sur des échantillons de peau prélevés au sein de la tache et au sein d'une zone adjacente non lésée. Les échantillons sont par exemple des biopsies de peau. Des biopsies de quelques millimètres de diamètres sont suffisantes, par exemple une biopsie de 2 mm, ou bien 3mm de diamètre. On peut envisager également l'excision totale d'une lésion.

Les niveaux d'expression des gènes de l'invention s'entendent des niveaux d'expression au sein des cellules du derme de la peau ou de l'échantillon sous étude.

La zone non lésée est de préférence une zone adjacente aussi proche que possible de la tache mais à une distance suffisante pour que la zone ou l'échantillon prélevé ne contienne aucune cellule susceptible d'appartenir à la tache pigmentaire. De préférence, la zone adjacente non lésée est une zone ayant une exposition à la lumière et au soleil comparable à la zone de la tache pigmentaire. Alternativement, la zone non lésée peut provenir d'une zone symétrique sur l'autre partie du sujet, ayant un emplacement parfaitement identique ; par exemple dans le cas d'une tache sur la main gauche, la zone non lésée peut être la zone correspondante sur la main droite. Dans ce cas, la zone non lésée n'est pas à proprement parler une zone adjacente. Par non lésée, on entend une zone qui ne possède pas de tache pigmentaire, ni d'irrégularité pigmentaire, de préférence une zone homogène en terme de pigmentation.

Du fait que la zone non lésée sert de référence, elle doit dans tous les cas être aussi comparable que possible que la zone de la tache, mais dépourvue de défaut pigmentaire.

Par niveau d'expression d'un gène, on entend préférentiellement, dans la présente description, le niveau de transcription dudit gène. Cependant, son niveau d'expression peut également se traduire par son niveau de traduction, à supposer toutefois qu'il s'agisse d'un gène codant pour une protéine. Tel est le cas pour les gènes de l'invention.

Concernant l'évaluation du niveau de transcription du gène choisi, elle peut être réalisée par différentes manières bien connues de l'homme du métier, directement ou bien après transcription inverse. Le niveau de transcription peut notamment être évalué par l'utilisation de puces à ARN ou puces à ADN vendues dans le commerce à cet effet. Une méthode d'évaluation possible est décrite dans la partie expérimentale.

Il est important de noter également que la méthode selon l'invention implique la comparaison des niveaux d'expression d'au moins l'un des gènes de la liste A ou de la liste B. De ce fait, il peut être suffisant d'évaluer quantitativement ou qualitativement la différence entre les deux niveaux d'expression, sans pour autant évaluer et quantifier individuellement chacun des niveaux d'expression.

Les niveaux d'expression d'au moins l'un des dits gènes peuvent être évalués par référence ou après normalisation avec le niveau d'expression d'autres gènes, dont le niveau d'expression est supposé sensiblement identique au sein de la tache et dans la zone de peau non lésée choisie. De tels gènes pour la normalisation sont bien connus de l'homme du métier et peuvent dépendre de la zone du corps où se trouve la tache. A titre d'exemple, les gènes suivants peuvent être cités comme susceptibles de servir à la normalisation des niveaux d'expression des gènes décrits, codant pour :
La protéine ribosomale L13a (RPL13A), la beta-2-microglobuline (B2M), la protéine ribosomale S9 (RPS9), la protéine ribosomale S28 (RPS28) et la Glyceraldehyde-3-phosphate dehydrogénase (GAPDH).

La méthode selon l'invention est pratiquée *in vitro* ou bien *ex vivo.*

Selon un mode de réalisation de la méthode de l'invention, la tache pigmentaire est une tache non pathologique, bénigne, notamment par opposition aux lésions pathologiques telles que les naevi ; il peut s'agir d'une irrégularité dans la pigmentation de la peau.

De préférence, une méthode telle que décrite comprend la comparaison des niveaux d'expression d'au moins deux gènes distincts parmi les gènes décrits, de préférence d'au moins trois gènes distincts, voire cinq gènes distincts. Il est également envisageable de comparer les niveaux d'expression d'au moins 6 gènes, voire d'au moins 8 gènes distincts ou bien de 10,12,15, 20 ou bien de tous les gènes décrits.

Selon une mise en oeuvre, les gènes distincts sont choisis au sein des gènes TGFBR2, TGFBI, BMP2, SMAD3, THBS2, TGFBR3, SEMA5A, SMAD7 et SOSTDC1, ou bien au sein de TGFBR2, TGFBI, BMP2, SMAD3, THBS2, TGFBR3, SMAD7 et SOSTDC1.

Quand deux gènes sont choisis, ils sont de préférence choisis parmi les listes des 6 gènes préférés suivants : TGFBR2, TGFBI, BMP2, SMAD3, THBS2 et TGFBR3. Par exemple, les gènes choisis peuvent être TGFBR2 et, TGFBI, ou bien TGFBR2 et BMP2, ou bien TGFBI et BMP2, ou bien TGFBR2 et TGFBR3. Toutes les combinaisons deux à deux, parmi les 6 gènes préférés, sont des combinaisons préférentielles pour la mise en oeuvre de l'invention. De même, toutes les combinaisons impliquant l'un des 6 gènes préférés et l'un des autres gènes de la liste A, sont particulièrement préférées.

Pour les combinaisons de 3 gènes, sont notamment envisagées les combinaisons suivantes ; TGFBR2, TGFBI et BMP2 ; TGFBR2, TGFBI et SMAD3 ; TGFBR2, TGFBI et TGFBR3 ; SMAD3, SEMA5A et SMAD7 ; TGFBR2, BMP2 et SOSTDC1 ; et TGFBI, BMP2 et SMAD3. Des combinaisons de 5 gènes envisageables selon la présente invention sont notamment les combinaisons suivantes : TGFBR2, TGFBI, BMP2, SMAD3 et THBS2; TGFBR2, TGFBI, BMP2, SMAD3 et TGFBR3 ; et TGFBR2, TGFBI, BMP2, THBS2 et TGFBR3.

Une combinaison particulière est celle comprenant les gènes TGFBR2, TGFBI, BMP2, SMAD3, THBS2, TGFBR3, SEMA5A et SMAD7, qui ont pour point commun d'être modulés de la même manière, notamment d'être surexprimés au sein de taches hyperpigmentaires, et d'être impliqués dans la voie de signalisation TGF-beta - SMAD. D'autres combinaisons sont également envisageables au sens de la présente invention, notamment des combinaisons comprenant au moins un gène parmi TGFBR2, TGFBI, THBS2 et TGFBR3; et au moins un gène parmi SMAD3, SEMA5A et SMAD7.

Les gènes TGFBR2, TGFBI, BMP2, SMAD3, THBS2, TGFBR3, SEMA5A, SMAD7 et SOSTDC1 ont pour point commun d'appartenir à la voie de signalisation TGF-β - SMAD et d'être modulés de façon coopérative au niveau de taches pigmentaires, c'est-à-dire que les gènes TGFBR2, TGFBI, BMP2, SMAD3, THBS2, TGFBR3, SEMA5A et SMAD7, qui sont positivement impliqués dans cette voie de signalisation sont tous modulés dans le même sens et ils sont modulés dans le sens inverse du gène SOSTDC1 qui est un antagoniste de BMP et donc négativement impliqué dans cette voie de signalisation.

Selon une autre mise en oeuvre, les gènes distincts sont choisis au sein des gènes de la liste B, ou bien parmi la liste des 6 gènes préférés suivants : FRAS1, LEPREL1, MATN2, DST, PLOD2 et ITGA2. Par exemple, les gènes choisis peuvent être FRAS1 et LEPREL1, ou bien FRAS1 et MATN2, ou bien LEPREL et MATN2, ou bien DST et PLOD2. Toutes les combinaisons deux à deux, parmi les 6 gènes préférés FRAS1, LEPREL1, MATN2, DST, PLOD2 et ITGA2, sont des combinaisons préférentielles pour la mise en oeuvre de l'invention.

De même, toutes les combinaisons impliquant l'un des 6 gènes préférés et l'un des autres gènes de la liste B sont particulièrement préférées.

Pour les combinaisons de 3 gènes, sont notamment envisagées les combinaisons suivantes : FRAS1, LEPREL1 et MATN2; FRAS1, LEPREL1 et DST; FRAS1, LEPREL1 et PLOD2 ; FRAS1, MATN2, et PLOD2 ; LEPREL1, MATN2 et PLOD2.

Des combinaisons de 5 gènes envisageables selon la présente invention sont notamment les combinaisons suivantes: FRAS1, LEPREL1, MATN2, DST et PLOD2; FRAS1, LEPREL1, MATN2, DST et ITGA2 ; FRAS1, LEPREL1, MATN2, PLOD2 et ITGA2.

Une combinaison particulière est celle constituée des gènes FRAS1, LEPREL1, MATN2, DST, ITGA2, COL6A3, CRTAP, LAMC1, LAMB3, LAMA3, ITGAV et ACTN1, qui ont pour point commun d'être modulés de la même manière, notamment d'être surexprimés au sein de taches hyperpigmentaires.

D'autres combinaisons sont également envisageables, notamment des combinaisons comprenant au moins un gène parmi ceux appartenant à la famille des collagènes, c'est-à-dire parmi LEPREL1, PLOD2, COL6A3 et CRTAP; au moins un gène parmi ceux appartenant à la famille des laminines, c'est-à-dire parmi LAMC1, LAMB3 et LAMA3 ; au moins un gène parmi ceux codant pour des protéines matricielles associées à la zone de la membrane basale, c'est-à-dire parmi FRAS1, MATN2 et DST ; au moins un gène de la famille des intégrines, c'est-à-dire parmi ITGA2 et ITGAV, et éventuellement ITGB1 ; et le gène ACTN1.

Alternativement, selon une mise en oeuvre préférée de l'invention, le gène dont le niveau d'expression est comparé entre la peau lésée et la peau saine, de préférence adjacente, est choisi parmi le sous-groupe constitué des gènes LEPREL1, PLOD2, COL6A3 et CRTAP. En effet, ces gènes ont pour point commun d'appartenir à la famille des collagènes, notamment à la famille des collagènes fibrillaires du stroma.

D'autres combinaisons sont également envisageables au sens de la présente invention, notamment des combinaisons comprenant au moins un gène parmi FRAS1, LEPREL1, MATN2, DST, ITGA2, COL6A3, CRTAP, LAMC1, LAMB3, LAMA3, ITGAV et ACTN1; et le gène PLOD2.

D'autres combinaisons sont envisagées, notamment des combinaisons comprenant au moins un gène parmi ceux impliqués dans la voie de signalisation TGF-béta - SMAD, c'est-à-dire parmi la liste A et au moins un gène parmi la liste B, et de préférence parmi FRAS1, LEPREL1, MATN2, DST, PLOD2 et ITGA2. Par exemple, une combinaison envisagée est celle constituée d'au moins deux gènes parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV et ACTN1 qui ont pour point commun d'être surexprimés au sein de taches hyperpigmentaires.

D'autres combinaisons sont notamment des combinaisons de 2 gènes, l'un provenant de la liste A, et l'autre provenant de la liste B, des combinaisons de 3 gènes, dont deux dans la liste A et un dans la liste B, ou l'inverse ; des combinaisons de 4 gènes avec 2 dans chacune des listes, ou bien trois dans la liste A et un dans la liste B, ou l'inverse.

Une combinaison de 2 gènes particulièrement préférée est la combinaison des gènes TGFBR2 et MATN2. Des combinaisons de 3 gènes préférées sont notamment la combinaison TGFBR2, BMP2 et MATN2 et la combinaison TGFBR2, MATN2 et LEPREL1. Des combinaisons de 4 gènes préférées sont notamment la combinaison TGFBR2, BMP2, SMAD3 et MATN2 ; la combinaison TGFBR2, MATN2, LEPREL1 et PLOD2 ; et la combinaison TGFBR2, BMP2, MATN2 et LEPREL1.

Toutes les combinaisons ou sous-groupes de gènes spécifiques préférés en rapport avec cet aspect de la description sont également préférés pour les autres aspects de la description.

La mise en oeuvre de la méthode décrite conduit à la conclusion que la tache supposée ou observée est une tache hyperpigmentaire si le niveau d'expression est
- supérieur dans la peau issue de la tache, ou dans l'échantillon de peau issue de la tache, par rapport au niveau dans la peau adjacente non lésée, ou dans l'échantillon de peau adjacente non lésée, si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et
- inférieur dans la peau issue de la tache, ou dans l'échantillon de peau issue de la tache, par rapport au niveau dans la peau adjacente non lésée, ou dans l'échantillon de peau adjacente non lésée, si le gène est choisi parmi SOSTDC1 et PLOD2.

En effet, les présents inventeurs ont mise en évidence la surexpression des gènes TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1 dans la peau issue de tache hyperpigmentaire, notamment de lentigo actinique, par rapport au niveau d'expression dans la peau adjacente non lésée. Ils ont également mis en évidence la sous-expression des gènes SOSTDC1 et PLOD2 dans la peau issue de tache hyperpigmentaire, notamment de lentigo actinique, par rapport au niveau d'expression dans la peau adjacente non lésée.

A l'inverse, la mise en oeuvre de la méthode décrite conduit à la conclusion que la tache supposée ou observée est une tache hypo-pigmentaire si le niveau d'expression est :
- inférieur dans la peau issue de la tache, ou dans l'échantillon de peau issue de la tache, par rapport au niveau dans la peau adjacente non lésée, ou dans l'échantillon de peau adjacente non lésée, si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et
- supérieur dans la peau issue de la tache, ou dans l'échantillon de peau issue de la tache, par rapport au niveau dans la peau adjacente non lésée, ou dans l'échantillon de peau adjacente non lésée, si le gène est choisi parmi SOSTDC1 et PLOD2.

Par supérieur ou inférieur, on entend une différence de niveaux d'expression qui est statistiquement significative, supérieure au bruit et reproductible. Par exemple, la différence de niveau d'expression est d'au moins 10 %, c'est-à-dire que si le niveau d'expression d'un gène de l'invention dans la peau non lésée est fixé à 1, le taux de modulation est d'au moins 1,1 pour un gène surexprimé dans la peau lésionnelle et d'au plus 0,9 pour un gène sous-exprimé dans la peau lésionnelle.

De préférence, la méthode décrite est mise en oeuvre avec au moins deux gènes, l'un appartenant à la catégorie des gènes surexprimés dans les taches hyperpigmentaires, et sous-exprimés dans les tache hypo-pigmentaires, et l'autre appartenant à la catégorie des gènes modulés à l'inverse du premier dans les taches pigmentaires. De préférence, la méthode est mise en oeuvre avec au moins trois gènes : deux gènes choisis parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et un gène choisi parmi SOSTDC1 et PLOD2, modulé à l'inverse des deux premiers dans les taches pigmentaires, ou bien deux gènes choisis parmi TGFBR2, TGFBI, BMP2, SMAD3, THBS2, TGFBR3, SEMA5A et SMAD7, et le gène SOSTDC1 ; ou bien deux gènes choisis parmi FRAS1, LEPREL1, MATN2, DST, ITGA2, COL6A3, CRTAP, LAMC1, LAMB3, LAMA3, ITGAV, ITGB1 et ACTN1 et le gène PLOD2, modulé à l'inverse des deux premiers dans les taches pigmentaires.

De préférence, dans le cadre de cette méthode de caractérisation, le niveau d'expression d'un des gènes décrits est comparé au sein de la peau d'un individu de type caucasien. De préférence, ledit individu est âgé d'au moins quarante ans, de préférence d'au moins cinquante ans, voire de soixante ans. L'individu considéré, dans le cadre de la présente invention, est de préférence de sexe féminin. Comme précédemment détaillé, le niveau d'expression d'un ou de plusieurs gènes décrits peut être comparé au sein d'échantillon de peau dudit individu.

Par ailleurs, les présents inventeurs ont également mis en évidence la modulation différentielle d'autres gènes dermiques entre de la peau issue d'une tache pigmentaire et de la peau non lésée, de préférence adjacente. Les inventeurs ont notamment mis en évidence la modulation des gènes dermiques suivants, liés à la matrice extracellulaire :
- Gènes EFEMP1, ECM1, ASPN, HS3ST6, PAPLN, CHSY1 et FLRT2 codant pour des protéines de la famille des protéoglycanes et glycoprotéines extracellulaires ;
- Gènes MXRA5, LYZ, CTSL2, PLAU et TIMP1 codant pour des protéines liées au remodelage matriciel.

Par conséquent, la méthode de caractérisation telle que décrite comprend également la comparaison des niveaux d'expression au sein de la peau issue de ladite tache et au sein de la peau non lésée, de préférence adjacente, d'au moins un gène dermique choisi parmi la liste constituée des gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1 et d'au moins un second gène choisi parmi la liste des gènes suivants : EFEMP1, ECM1, ASPN, HS3ST6, PAPLN, CHSY1, FLRT2, MXRA5, LYZ, CTSL2, PLAU et TIMP1; par exemple le second gène est choisi parmi la liste des gènes suivants EFEMP1, ECM1, ASPN, HS3ST6, PAPLN, CHSY1, FLRT2 ou bien parmi la liste des gènes suivants : MXRA5, LYZ, CTSL2, PLAU et TIMP1.

Des gènes préférés au sein de la liste de gènes dermiques EFEMP1, ECM1, ASPN, HS3ST6, PAPLN, CHSY1, FLRT2 sont les gènes EFEMP1, ECM1, ASPN, HS3ST6, PAPLN et CHSY1 ; des gènes préférés sont aussi les gènes MXRA5, LYZ, CTSL2, PLAU et TIMP1.

De préférence, le second gène est choisi parmi les gènes EFEMP1, ECM1, ASPN, HS3ST6, PAPLN, CHSY1 et FLRT2, et préférentiellement parmi les gènes EFEMP1, ECM1, ASPN, HS3ST6, PAPLN et CHSY1. Alternativement, le second gène est choisi parmi les gènes MXRA5, LYZ, CTSL2, PLAU et TIMP1.

Selon une autre mise en oeuvre, le premier gène est choisi parmi la liste A de gènes dermiques impliqués dans la voie de signalisation TGF-β - SMAD ; le second gène est choisi comme précisé ci-dessus. Selon encore une autre mise en oeuvre, le premier gène est choisi parmi la liste B; le second gène étant choisi comme précisé ci-dessus.

Selon une mise en oeuvre particulière, la méthode comprend la comparaison des niveaux d'expression d'au moins 3 gènes distincts, l'un choisi parmi les gènes TGFBR2, TGFBI, BMP2, SMAD3, THBS2, TGFBR3, SEMA5A, SMAD7 et SOSTDC1, un second choisi parmi les gènes EFEMP1, ECM1, ASPN, HS3ST6, PAPLN, CHSY1 et FLRT2 et un troisième choisi parmi les gènes MXRA5, LYZ, CTSL2, PLAU et TIMP1. Alternativement, quand 3 gènes distincts sont choisis, le premier peut être choisi parmi les gènes de la liste B, le second et le troisième étant choisi comme décrit ci-dessus. Selon encore une autre alternative, le premier gène est choisi parmi la liste des gènes comprenant TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1 ; éventuellement SEMA5A est toutefois exclu.

Selon une autre mise en oeuvre encore, la méthode comprend la comparaison des niveaux d'expression d'au moins 4 gènes distincts, les trois premiers étant choisis comme décrit précédemment et le 4^{ème} dans la liste constituée de FRAS1, LEPREL1, MATN2, DST, PLOD2, ITGA2, COL6A3, CRTAP, LAMC1, LAMB3, LAMA3, ITGAV, ITGB1 et ACTN1.

Toutes les associations de gènes décrites ci-dessus sont également des associations préférées dans le cadre des autres aspects de la description.

Si un gène supplémentaire, ou plusieurs, est choisi parmi les gènes EFEMP1, ECM1, ASPN, HS3ST6, PAPLN, CHSY1, FLRT2, MXRA5, LYZ, CTSL2, PLAU et TIMP1, alors la méthode de caractérisation conduit à confirmer un tache hyperpigmentaire si le niveau d'expression est
- supérieur dans la peau issue de la tache, ou dans l'échantillon de peau issue de la tache, par rapport au niveau dans la peau adjacente non lésée, ou dans l'échantillon de peau adjacente non lésée, si le gène est choisi parmi EFEMP1, ASPN, PAPLN, CHSY1, MXRA5, LYZ, PLAU et TIMP1, et
- inférieur dans la peau issue de la tache, ou dans l'échantillon de peau issue de la tache, par rapport au niveau dans la peau adjacente non lésée, ou dans l'échantillon de peau adjacente non lésée, si le gène est choisi parmi HS3ST6, FLRT2, ECM1 et CTSL2.

A l'inverse, la méthode d'évaluation conduit à confirmer une tache hypo-pigmentaire si le niveau d'expression est :
- inférieur dans la peau issue de la tache, ou dans l'échantillon de peau issue de la tache, par rapport au niveau dans la peau adjacente non lésée, ou dans l'échantillon de peau adjacente non lésée, si le gène est choisi parmi EFEMP1, ASPN, PAPLN, CHSY1, MXRA5, LYZ, PLAU et TIMP1, et
- supérieur dans la peau issue de la tache, ou dans l'échantillon de peau issue de la tache, par rapport au niveau dans la peau adjacente non lésée, ou dans l'échantillon de peau adjacente non lésée, si le gène est choisi parmi HS3ST6, FLRT2, ECM1 et CTSL2.

La méthode décrite est également une méthode de test pour la prédiction de la formation de taches cutanées chez un sujet. Selon cette mise en oeuvre, le niveau d'expression d'au moins un, et de préférence plusieurs, gène de l'invention est comparé dans un échantillon de peau, à son niveau d'expression dans la peau normale. Une modulation significative du niveau d'expression par rapport à la peau normale permet de conclure que la peau du sujet testé est encline à la formation de taches cutanées.

Par peau normale, il peut s'agir ou bien de la peau du même sujet, dans une zone corporelle notoirement dépourvue de taches, par exemple des zones non exposées au rayonnement solaire, ou bien des zones peu enclines à la formation de taches pigmentaires. Il peut également s'agir du niveau d'expression moyen desdits gènes, dans la peau de personnes dépourvues de taches et ayant de préférence le même type de peau que le sujet testé. Les gènes de normalisation décrits plus haut pourront être utilisés pour normaliser les taux d'expression des gènes de l'invention.

La présente invention concerne également, selon un second aspect, une méthode d'évaluation de l'efficacité d'un traitement des lentigos actiniques selon la revendication 5, utilisant la signature mise en évidence par les inventeurs. Le traitement évalué peut être un traitement visant l'atténuation de tache pigmentaire ou toute autre modulation de la pigmentation, pour notamment uniformiser le teint, homogénéiser la couleur de la peau ou lutter contre les dyschromies. Selon une première mise en oeuvre, cette méthode d'évaluation comprend une étape de comparaison des niveaux d'expression dans la peau issue d'une tache pigmentaire, avant et après traitement, d'au moins un gène dermique choisi parmi les gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1.

Selon une mise en oeuvre, le gène est choisi parmi TGFBR2, TGFBI, BMP2, SMAD3, THBS2, TGFBR3, SEMA5A, SMAD7 et SOSTDC1, ou bien parmi TGFBR2, TGFBI, BMP2, SMAD3, THBS2, TGFBR3, SMAD7 et SOSTDC1. Selon une autre mise en oeuvre, le gène est choisi parmi FRAS1, LEPREL1, MATN2, DST, PLOD2, ITGA2, COL6A3, CRTAP, LAMC1, LAMB3, LAMA3, ITGAV, ITGB1 et ACTN1. SEMA5A et/ou ITGB1 sont éventuellement exclus des listes.

Selon cette méthode d'évaluation, la comparaison des niveaux d'expression du ou des gènes choisis est réalisée au sein de la peau issue d'une tache de vieillesse, ou bien au sein d'un échantillon correspondant, avant et après traitement. Il n'y a donc pas de comparaison à un niveau d'expression au sein de peau saine non lésée.

Comme pour la méthode de caractérisation selon le premier aspect de l'invention, les niveaux d'expression sont avantageusement normalisés à l'aide des niveaux d'expression des gènes codant pour la protéine ribosomale L13a (RPL13A), la beta-2-microglobuline (B2M), la protéine ribosomale S9 (RPS9), la protéine ribosomale S28 (RPS28) et la Glyceraldehyde-3-phosphate dehydrogénase (GAPDH).

Par la méthode d'évaluation telle que décrite, un traitement donné est considéré comme efficace pour le traitement d'une tache hyperpigmentaire si le niveau d'expression est :
- inférieur après traitement par rapport au niveau d'expression avant traitement, si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et
- supérieur après traitement par rapport au niveau d'expression avant traitement, si le gène est choisi parmi SOSTDC1 et PLOD2.

A l'inverse, par la méthode d'évaluation de l'invention, un traitement donné est considéré comme efficace pour le traitement de taches hypo-pigmentaires quand le niveau d'expression est :
- supérieur après traitement par rapport au niveau d'expression avant traitement, si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et
- inférieur après traitement par rapport au niveau d'expression avant traitement, si le gène est choisi parmi SOSTDC1 et PLOD2.

Un traitement sera considéré comme sans effet si les niveaux d'expression du gène choisi, avant et après traitement, sont sensiblement identiques, ou bien si les différences observées ne sont pas significatives.

Quand il est comparé les niveaux d'expression de plus d'un gène, le traitement est considéré comme efficace pour le traitement d'une tache ou d'une irrégularité pigmentaire si, pour la majorité des gènes testés, et de préférence pour l'ensemble des gènes testés, pris individuellement, le traitement est considéré comme efficace. Pour les autres gènes choisis, le traitement doit de préférence être sans effet, mais non avoir l'effet inverse.

Selon une autre mise en oeuvre, la méthode d'évaluation de l'efficacité d'un traitement des taches pigmentaires cutanées comprend la caractérisation d'une tache ou irrégularité pigmentaire selon la méthode décrite, avant et après traitement, et la comparaison des différences de niveaux d'expression observées entre la peau lésée de la tache ou irrégularité pigmentaire et la peau saine.

Selon cette mise en oeuvre de l'évaluation de l'efficacité d'un traitement, le traitement est considéré comme efficace si la différence entre les niveaux d'expression du ou des gènes choisis dans la peau lésée issue de la tache par rapport à la peau saine, de préférence adjacente, est moindre après traitement par rapport à ce qu'elle était avant le traitement.

Quand il est comparé les niveaux d'expression de plus d'un gène, il est de préférence conclu à l'efficacité du traitement quand pour la majorité des gènes choisis, et de préférence pour l'ensemble des gènes choisis, pris individuellement, on peut conclure à un traitement efficace.

De préférence, la comparaison des niveaux d'expression du ou des gènes choisis est réalisée sur des échantillons de peau prélevés à partie de la tache pigmentaire.

Le traitement considéré, évalué par la méthode de l'invention, n'est pas limité à un type particulier de traitement. Il peut s'agit d'un traitement par une molécule chimique, un actif, un extrait naturel, notamment une huile essentielle, un acide nucléique, notamment un ARNi, un complexe protéique ou tout autre molécule ou combinaison de molécules. Il peut également s'agir d'un traitement par un moyen physique ou des ondes, notamment électromagnétiques. Il s'agit de préférence d'un traitement topique, mais il est également envisagé d'évaluer l'efficacité de traitements administrés par voie orale, par injection, ou par tout autre moyen d'administration.

Le traitement testé peut viser à atténuer ou faire disparaitre une tache cutanée, à moduler la pigmentation de la peau, à uniformiser le teint, à homogénéiser la couleur de la peau ou à atténuer les dyschromies.

Des traitements particulièrement préférés dans le cadre de cette invention sont des traitements cosmétiques, plus particulièrement des traitements topiques cosmétiques. Dans ce cas, la tache pigmentaire considérée est un lentigo actinique, solaire ou sénile.

Par les méthodes de l'invention, il est également possible d'évaluer l'efficacité de la combinaison de plusieurs traitements. Il est en effet possible d'évaluer des combinaisons permettant au mieux de restaurer les niveaux d'expression d'un, de plusieurs ou de tous les gènes de l'invention, ou de la liste A ou de la liste B, tels qu'ils sont exprimés au sein de peau non lésée.

Par les méthodes d'évaluation décrites ci-dessus, il est possible d'évaluer l'efficacité d'un nouveau traitement envisagé, ou bien également de quantifier ou qualifier l'efficacité de traitements déjà existants contre les taches pigmentaires, qu'elles soient hyperpigmentaires ou bien hypo-pigmentaires. Par ce biais, il peut aussi être envisagé des combinaisons de traitements susceptibles d'être particulièrement efficaces, synergiques ou complémentaires. Comme explicité pour les méthodes de caractérisation selon le premier aspect de l'invention, il est de préférence comparé les niveaux d'expression de plus d'un gène, de préférence d'au moins deux, trois, cinq, six, huit, dix, douze, quinze, dix-huit ou bien de tous les gènes de l'invention, ou bien des gènes de la liste A, ou bien des gènes de la liste B.

Des gènes ou des combinaisons de gènes tout particulièrement préférés ont déjà été explicités en regard du premier aspect de l'invention ; les mêmes gènes ou combinaisons sont préférés dans cet aspect. Notamment, il est tout particulièrement préféré que le gène choisi le soit parmi les gènes TGFBR2, TGFBI, BMP2, SMAD3, THBS2, TGFBR3, SEMA5A, SMAD7 et SOSTDC1, et tout particulièrement parmi les gènes TGFBR2, TGFBI, BMP2, SMAD3, THBS2 et TGFBR3, et toutes les combinaisons deux à deux ou trois à trois des gènes de ces listes sont particulièrement préférées. Selon une autre mise en oeuvre, le gène est choisi parmi les gènes de la liste B, et notamment parmi la liste des gènes FRAS1, LEPREL1, MATN2, DST, PLOD2 et ITGA2, et toutes les combinaisons deux à deux ou trois à trois des gènes de cette liste sont particulièrement préférées.

Par ailleurs, du fait des résultats complémentaires obtenus par les inventeurs concernant d'autres gènes dermiques dont le niveau d'expression est modulé au sein de tache pigmentaire, les méthodes d'évaluation telles que décrites sont de préférence mises en oeuvre avec :
- au moins un gène choisi parmi les gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1, et même de préférence parmi les gènes de la liste A ou parmi les gènes TGFBR2, TGFBI, BMP2, SMAD3, THBS2 et TGFBR3, ou parmi les gènes de la liste B, ou encore parmi les gènes FRAS1, LEPREL1, MATN2, DST, PLOD2 et ITGA2 et
- au moins un second gène choisi parmi les gènes EFEMP1, ECM1, ASPN, HS3ST6, PAPLN, CHSY1, FLRT2, MXRA5, LYZ, CTSL2, PLAU et TIMP1. Alternativement, le second gène peut être choisi parmi les gènes EFEMP1, ECM1, ASPN, HS3ST6,

PAPLN, CHSY1, FLRT2 ou bien parmi les gènes MXRA5, LYZ, CTSL2, PLAU et TIMP1. Des gènes préférés au sein des gènes dermiques EFEMP1, ECM1, ASPN, HS3ST6, PAPLN, CHSY1, FLRT2 sont les gènes EFEMP1, ECM1, ASPN, HS3ST6, PAPLN et CHSY1.

Si un gène supplémentaire, ou plusieurs, est choisi parmi les gènes EFEMP1, ECM1, ASPN, HS3ST6, PAPLN, CHSY1, FLRT2, MXRA5, LYZ, CTSL2, PLAU et TIMP1, alors la méthode d'évaluation conduit à considérer un traitement efficace pour le traitement de taches hyperpigmentaires si le niveau d'expression est
- inférieur après traitement par rapport au niveau d'expression avant traitement, si le gène est choisi parmi EFEMP1, ASPN, PAPLN, CHSY1, MXRA5, LYZ, PLAU et TIMP1, et
- supérieur après traitement par rapport au niveau d'expression avant traitement, si le gène est choisi parmi HS3ST6, FLRT2, ECM1 et CTSL2.

A l'inverse, la méthode d'évaluation conduit à considérer un traitement efficace pour le traitement de taches hypo-pigmentaires si le niveau d'expression est :
- supérieur après traitement par rapport au niveau d'expression avant traitement, si le gène est choisi parmi EFEMP1, ASPN, PAPLN, CHSY1, MXRA5, LYZ, PLAU et TIMP1, et
- inférieur après traitement par rapport au niveau d'expression avant traitement, si le gène est choisi parmi HS3ST6, FLRT2, ECM1 et CTSL2.

L'échantillon de peau provient de préférence d'un être humain de type caucasien, de préférence âgé d'au moins quarante ans, de préférence d'au moins cinquante ans, voire au moins soixante ans.

De préférence, dans le cadre de la présente invention et notamment pour la méthode d'évaluation décrite, il s'agit de traitement de taches hyperpigmentaires de type lentigos actiniques, solaires ou séniles.

La méthode d'évaluation de l'efficacité d'un traitement selon la présente invention est remise en oeuvre in vitro ou ex vivo.

Il est souhaitable que l'échantillon de peau avant traitement et après traitement soit prélevé à partir de la même tache pigmentaire, ou bien d'une tache pigmentaire très proche si la taille de la tache ne permet pas d'obtenir aisément des échantillons avant et après traitement.

La présente invention concerne également une méthode *in vitro* d'évaluation de l'efficacité d'un traitement des lentigos actiniques; une telle méthode comprend la comparaison, avant et après traitement, du niveau d'expression dans un modèle cellulaire représentatif de la peau, d'au moins un gène dermique choisi parmi la liste constituée des gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1, ou bien du niveau d'expression ou d'activité du produit d'expression dudit gène choisi. Alternativement, le gène peut être choisi parmi la liste A de gènes dermiques liés à la voie de signalisation TGF-béta - SMAD, avec ou sans SEMA5A, ou bien parmi la liste B, ou bien parmi les différents sous-groupes de cette dernière liste, à savoir parmi LEPREL1, PLOD2, COL6A3 et CRTAP, ou bien parmi LAMC1, LAMB3 et LAMA3, ou bien parmi FRAS1, MATN2 et DST, ou bien encore parmi ITGA2, ITGAV et ITGBL, ou entre ITGA2 et ITGAV.

Le modèle cellulaire peut être de tout type considéré comme approprié par l'homme du métier. Il peut s'agir notamment de modèles cellulaires mono ou co-cultures, ou bien de modèles tridimensionnels de peau reconstruite, ou bien de peau cultivée ex-vivo. Il existe également des modèles cellulaires représentatifs de taches pigmentaires, plus particulièrement de lentigos actiniques, qui peuvent être utilisés dans le cadre de cette méthode. De tels modèles cellulaires n'ont pas besoin de mimer les taches pigmentaires (ou le lentigo) dans leur globalité mais doivent mimer des évènements biologiques, des caractéristiques morphologiques ou pigmentaires observés dans les taches pigmentaires, notamment le lentigo. De tels modèles in vitro sont bien connus de l'homme du métier.

Selon une mise en oeuvre préférée de la méthode in vitro décrite ci-dessus, elle comprend la comparaison des niveaux d'expression d'au moins deux gènes, de préférence d'au moins trois, cinq ou six gènes de l'invention, comme explicité pour les autres méthodes d'évaluation selon l'invention, lesdits gènes étant choisis parmi les gènes de l'invention, ou bien parmi les gènes de la liste A, avec ou sans SEMA5A, ou bien parmi les gènes de la liste B, avec ou sans ITGB1. Des gènes tout particulièrement préférés sont les gènes TGFBR2, TGFBI, BMP2, SMAD3, THBS2 et TGFBR3, ainsi que les gènes FRAS1, LEPREL1, MATN2, DST, PLOD2 et ITGA2.

De même, comme explicité pour les autres méthodes d'évaluation, elles sont de préférence mises en oeuvre avec au moins deux gènes,
- l'un étant choisi parmi les gènes TGFBR2, TGFBI, BMP2, SMAD3, THBS2, TGFBR3, SEMA5A, SMAD7 et SOSTDC1, ou bien parmi les gènes de la liste B, ou bien encore parmi TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1, et
- le second étant choisi parmi les gènes EFEMP1, ECM1, ASPN, HS3ST6, PAPLN, CHSY1, FLRT2, MXRA5, LYZ, CTSL2, PLAU et TIMP1, de préférence parmi les gènes EFEMP1, ECM1, ASPN, HS3ST6, PAPLN, CHSY1 et FLRT2 ou bien parmi les gènes MXRA5, LYZ, CTSL2, PLAU et TIMP1.

D'une manière particulièrement préférée, le premier gène est choisi parmi TGFBR2, TGFBI, BMP2, SMAD3, THBS2 et TGFBR3 ou bien parmi FRAS1, LEPREL1, MATN2, DST, PLOD2 et ITGA2.

Concernant les méthodes d'évaluation selon l'invention, selon une mise en oeuvre préférée, le premier gène est choisi parmi le sous-groupe constitué des gènes LEPREL1, PLOD2, COL6A3 et CRTAP, appartenant à la famille des collagènes, notamment à la famille des collagènes fibrillaires du stroma.

Selon une autre mise en oeuvre, le premier gène est choisi parmi le groupe constitué des gènes LAMC1, LAMB3 et LAMA3, codant pour des lamines et étant surexprimés au niveau de taches hyperpigmentaires.

Selon une autre mise en oeuvre, le premier gène est choisi parmi le groupe constitué des gènes FRAS1, MATN2 et DST, codant pour des protéines matricielles associées à la zone de la membrane basale et étant surexprimés au niveau de taches hyperpigmentaires.

Selon encore une autre mise en oeuvre, le premier gène est choisi parmi le groupe constitué des gènes ITGA2, ITGAV et ITGB1, codant pour des intégrines et étant surexprimés au niveau de taches hyperpigmentaires.

Alternativement, la méthode peut être mise en oeuvre avec le gène ACTN1, codant pour une actine.

Toutes les combinaisons de gènes spécifiques décrites en regard des autres aspects de la description sont également préférées dans le cadre de cet aspect.

Par ailleurs, au moyen de ces méthodes d'évaluation, il est possible de promouvoir un traitement auprès de consommateurs, en mettant en avant les résultats obtenus avec ce traitement dans les méthodes d'évaluation de l'efficacité décrites dans la présente invention.

Une méthode permettant de recommander un produit en signalant son effet dans un protocole de test constitué par une méthode d'évaluation de l'efficacité telle que décrite précédemment est également décrite, par exemple une méthode pour la promotion d'un produit cosmétique ou traitement cosmétique consistant à faire état d'une efficacité, action ou propriété dudit produit ou traitement, démontrée par au moins une méthode opérée telle que décrite précédemment.

Une telle promotion du produit pourra se faire par n'importe quel canal de communication. Elle pourra être faite notamment par le vendeur ou la vendeuse, directement sur le point de vente, par la radio et la télévision, notamment dans le cadre de spots publicitaires. Elle pourra être faite également par le canal de la presse écrite, ou par le biais de tout autre document, en particulier à des fins publicitaires (prospectus). Elle pourra se faire également par Internet, ou par tout autre réseau informatique adéquat. Elle pourra être faite également directement sur le produit, notamment sur son packaging ou sur toute notice explicative qui peut lui être associée.

La présente description concerne également une méthode de criblage de molécules pour le traitement de taches pigmentaires cutanées comprenant la mise en oeuvre d'une des méthodes d'évaluation décrites précédemment pour déterminer l'efficacité d'un traitement à base de cette molécule.

Selon un autre aspect, la présente invention concerne également une méthode cosmétique de traitement ou de prévention de lentigo actinique non pathologique de la peau humaine, comprenant la modulation du niveau d'expression ou de l'activité d'un gène dermique, où ledit gène est choisi parmi la liste constituée des gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV et ACTN1. Alternativement, le gène peut être choisi parmi la liste A de gènes dermiques liés à la voie de signalisation TGF-béta - SMAD, de préférence sans SEMA5A, ou bien parmi la liste des gènes dermiques B, de préférence sans ITGB1, ou bien parmi les différents sous-groupes de cette dernière liste, à savoir parmi LEPREL1, PLOD2, COL6A3 et CRTAP, ou bien parmi LAMC1, LAMB3 et LAMA3, ou bien parmi FRAS1, MATN2 et DST, ou bien encore parmi ITGA2, ITGAV et ITGB1, ou entre ITGA2 et ITGAV.

Les lentigos actiniques non pathologiques s'entendent de taches bénignes, dont l'élimination n'est souhaitable que pour des raisons esthétiques et nullement pour des raisons thérapeutiques.

Les présents inventeurs ont montré le rôle important des gènes de l'invention au sein du derme au niveau des taches pigmentaires, et notamment le lien entre dérégulation du niveau d'expression de ces gènes et apparition de taches pigmentaires. De ce fait, suspendre ou diminuer la modulation de ces gènes peut permettre de diminuer ou abolir les dérégulations observées et ainsi permettre de restaurer une situation au niveau de la matrice extracellulaire compatible avec l'absence de tache pigmentaire, avec donc un teint uniforme et une couleur de peau homogène.

Pour les mêmes raisons qu'explicitées en regard des autres aspects de l'invention, il est de préférence choisi plus d'un gène au sein des gènes de l'invention, par exemple au moins deux gènes, au moins trois, cinq, six, huit. Selon une mise en oeuvre, la méthode cosmétique a pour but de moduler le niveau d'expression de l'intégralité des gènes de l'invention, ou bien de l'intégralité des gènes de la liste A, avec ou sans SEMA5A, ou bien de l'intégralité des gènes de la liste B. De préférence, le gène ITGB1 est alors exclu de la liste B. La méthode cosmétique selon l'invention a pour but de restaurer des niveaux d'expression proches de ceux qui sont observés au sein de la peau saine, par exemple adjacente d'une tache pigmentaire. Des gènes particulièrement préférés sont les gènes TGFBR2, TGFBI, BMP2, SMAD3, THBS2 et TGFBR3, ainsi que les gènes FRAS1, LEPREL1, MATN2, DST, PLOD2 et ITGA2. Dans le cas d'un lentigo actinique, sénile ou solaire, la modulation recherchée dans les méthodes cosmétiques selon l'invention est :
- une inhibition, totale, partielle ou temporaire, de l'expression d'au moins un gène choisi parmi les gènes TGFBR2, TGFBI, BMP2, SMAD3, THBS2, TGFBR3, SEMA5A et SMAD7, ou bien parmi les gènes FRAS1, LEPREL1, MATN2, DST, ITGA2, COL6A3, CRTAP, LAMC1, LAMB3, LAMA3, ITGAV, ITGB1 et ACTN1, ou bien parmi les gènes TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1 ou bien
- une augmentation, éventuellement temporaire de l'expression du gène SOSTDC1, ou du gène PLOD2.

De préférence, l'inhibition n'est pas une inhibition totale, mais partielle, tendant à diminuer le niveau d'expression du gène choisi sans pour autant inhiber entièrement son expression.

Décrit également est le cas d'une tache hypopigmentaire. Dans un tel cas, la modulation recherchée est inverse par rapport à la situation précédente, notamment une telle méthode vise à augmenter l'expression d'au moins un gène choisi parmi les gènes TGFBR2, TGFBI, BMP2, SMAD3, THBS2, TGFBR3, SEMA5A et SMAD7, ou bien parmi les gènes FRAS1, LEPREL1, MATN2, DST, ITGA2, COL6A3, CRTAP, LAMC1, LAMB3, LAMA3, ITGAV, ITGB1 et ACTN1, ou bien parmi les gènes TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, ou bien à inhiber ou diminuer le niveau d'expression du gène SOSTDC1 ou du gène PLOD2.

Par augmentation ou diminution du niveau d'expression, il est inclus l'augmentation ou la diminution du niveau de transcription desdits gènes, et l'augmentation ou la diminution du niveau de traduction desdits gènes, ainsi que l'augmentation ou la diminution de l'activité des protéines codées par ces gènes.

De préférence, concomitamment à la modulation du niveau d'expression recherchée pour l'un des gènes de l'invention, une méthode cosmétique selon l'invention comprend de préférence également la modulation d'au moins un autre gène dermique, choisi parmi les gènes EFEMP1, ECM1, ASPN, HS3ST6, PAPLN, CHSY1, FLRT2, MXRA5, LYZ, CTSL2 et PLAU et TIMP1. Des gènes préférés au sein de cette seconde liste de gènes dermiques sont les gènes EFEMP1, ECM1, ASPN, HS3ST6, PAPLN et CHSY1 et les gènes MXRA5, LYZ, CTSL2, PLAU et TIMP1.

Les modulations appliquées, s'il s'agit du traitement d'une tache hyperpigmentaire sont la diminution du niveau d'expression pour les gènes EFEMP1, ASPN, PAPLN, CHSY1, MXRA5, LYZ, PLAU et TIMP1, et l'augmentation du niveau d'expression pour les gènes ECM1, HS3ST6, FLRT2 et CTSL2. Dans le cas de traitement de tache hypo-pigmentaire, les modulations appliquées sont inverses.

Une méthode cosmétique selon la présente invention comprend donc l'application d'un produit, notamment molécule chimique, extrait naturel, actif, acides nucléiques, peptides, ou d'un traitement modulant le niveau d'expression ou l'activité du produit d'expression d'au moins un des gènes dermiques de l'invention.

Les modulations sont de préférence obtenues grâce à l'utilisation d'un antisens, d'un microARN ou d'un siRNA dirigé contre au moins un des gènes de l'invention et inhibant son expression.

S'il s'agit d'un produit, il est de préférence appliqué de façon topique.

De préférence, la modulation est effectuée grâce à l'utilisation d'un modulateur d'un des gènes de l'invention. De tels modulateurs sont décrits dans l'exemple 3 et le tableau 3. Par exemple, une méthode cosmétique telle que décrite comprendra avantageusement l'application d'un composé choisi parmi la poudre osseuse déminéralisée ou « Demineralized bone powder (DBP) », le Pioglitazone, GW0742, le Cristata L flavonoid, le Fénofibrate, l'Oxymatrine, l'acide salvianolique B, le SB-431542, un extrait de Wen-pi-tang-Hab-Wu-ling-san, le Tetrandrine et la N-acetyl-seryl-aspartyl-lysyl-proline (Ac-SDKP), la vitamine K2 menaquinone, le beta-aminopropionitrile (bAPN) et le Vandetanib (ZD6474 5), ou bien l'application d'ultrasons pulsés de faible intensité, ou bien encore parmi les associations d'au moins deux de ces modulateurs. Par ailleurs, une méthode cosmétique est avantageusement mise en oeuvre après la caractérisation de la tache pigmentaire que l'on souhaite traiter par une méthode selon le premier aspect. En effet, cette première étape permet de caractériser la tache et donc de détecter les gènes dont le niveau d'expression est fortement modulé entre la zone de la tache et une zone non lésée, de préférence adjacente. Il est ensuite possible d'adapter un traitement qui permette d'agir sur le ou les gènes de l'invention qui sont modulés différentiellement au sein de cette tache, en appliquant spécifiquement des modulateurs pour lesdits gènes.

Quel que soit le traitement considéré, la méthode cosmétique peut également comprendre l'application d'un ou plusieurs composés actifs additionnels visant à renforcer les effets recherchés par exemple, toute substance décrite comme dépigmentante, des agents kératolytiques et/ou desquamants, des antioxydants, des filtres solaires chimiques ou physiques UV, des agents anti-inflammatoires et/ou apaisants, des acides désoxyribonucléiques et leurs dérivés.

La méthode cosmétique est également applicable dans le cas de la prévention de l'apparition de taches pigmentaires ou d'autres irrégularités du teint ou de la couleur de la peau, et notamment de taches hyperpigmentées telles que le lentigo actinique.

En effet, les différentes données obtenues par les inventeurs et détaillées dans la partie expérimentale révèlent que les atteintes à la matrice extracellulaire et notamment à la voie de signalisation TGF-β - SMAD, à travers les gènes mis en évidence par les inventeurs sont susceptibles de survenir en amont, avant l'apparition des taches. On peut penser que la séquence des évènements biologiques dans les taches hyperpigmentaires puisse être la suivante : 1) altération du derme (du fait de la modulation de l'expression des gènes identifiés dans cette demande) qui conduit à 2) modification de l'épiderme avec formation de crête épidermique dans le derme qui amène à 3) une augmentation de la longueur de la jonction dermo-épidermique et la formation de réseaux complexes conduisant à 4) une augmentation de la charge mélanique.

La présente invention concerne également l'utilisation d'un modulateur du niveau d'expression ou de l'activité du produit d'expression d'au moins un gène dermique choisi parmi la liste constituée des gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV, ITGB1 et ACTN1, pour une application cosmétique dans le traitement de lentigos actiniques non-pathologiques, ledit modulateur étant un antisens, un microARN ou un siRNA dirigé contre le gène dermique choisi et inhibant l'expression dudit gène.

Alternativement, le gène peut être choisi parmi la liste A de gènes dermiques liés à la voie de signalisation TGF-béta - SMAD, avec ou sans SEMA5A, ou bien parmi la liste B de gènes dermiques, ou bien parmi les différents sous-groupes de cette dernière liste, à savoir parmi LEPREL1, PLOD2, COL6A3 et CRTAP, ou bien parmi LAMC1, LAMB3 et LAMA3, ou bien parmi FRAS1, MATN2 et DST, ou bien encore parmi ITGA2, ITGAV et ITGB1, ou entre ITGA2 et ITGAV. ITGB1 peut être exclu des différentes listes.

Pour le traitement de taches hyperpigmentaires, le modulateur dont il est fait usage est un inhibiteur d'au moins un gène choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1 ou bien un activateur du gène SOSTDC1 ou du gène PLOD2. De préférence, l'inhibiteur est un inhibiteur partiel conduisant à la diminution du niveau d'expression ou à la diminution de l'activité du produit d'expression dudit gène, sans pour autant abolir entièrement l'expression ou l'activité de ce gène. Le modulateur est un inhibiteur d'au moins un gène choisi parmi TGFBR2, TGFBI, BMP2, SMAD3, THBS2, TGFBR3, SEMA5A et SMAD7, ou sans SEMA5A, ou bien un activateur de SOSTDC1. Le modulateur peut aussi être un inhibiteur d'au moins un gène choisi parmi FRAS1, LEPREL1, MATN2, DST, ITGA2, COL6A3, CRTAP, LAMC1, LAMB3, LAMA3, ITGAV et ACTN1, ou bien un activateur de PLOD2.

A l'inverse, pour le traitement de taches hypopigmentaires, le modulateur dont il est fait usage est un activateur d'au moins un gène choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, ou bien un inhibiteur du gène SOSTDC1 ou du gène PLOD2. Le modulateur est un activateur d'au moins un gène choisi parmi TGFBR2, TGFBI, BMP2, SMAD3, THBS2, TGFBR3, SEMA5A et SMAD7, ou sans SEMA5A, ou bien un inhibiteur de SOSTDC1. Dans un autre cas, le modulateur est un activateur d'au moins un gène choisi parmi FRAS1, LEPREL1, MATN2, DST, ITGA2, COL6A3, CRTAP, LAMC1, LAMB3, LAMA3, ITGAV et ACTN1, ou bien un inhibiteur de PLOD2.

Des exemples d'inhibiteurs ou d'activateurs déjà connus et caractérisés sont divulgués dans l'exemple 3 de la partie expérimentale. De tels modulateurs peuvent notamment être l'acide salvianolique B, l'extrait Wen-pi-tang-Hab-Wu-ling-san ou des ultra-sons pulsés de faible intensité, pour un usage dans le traitement cosmétique de taches hyperpigmentaires.

Des modulateurs bien connus sont également des molécules antisens, des siRNAs, et des microARNs. Des modulateurs envisagés sont notamment des antisens, des microARNs et des siRNAs dirigés contre au moins un des gènes de l'invention et inhibant son expression.

Des modulateurs sont notamment la poudre osseuse déminéralisée ou « Demineralized bone powder (DBP) », le Pioglitazone, GW0742, le Cristata L flavonoid, le Fénofibrate, l'Oxymatrine, l'acide salvianolique B, le SB-431542, un extrait de Wen-pi-tang-Hab-Wu-ling-san, le Tetrandrine, la N-acetyl-seryl-aspartyl-lysyl-proline (Ac-SDKP), la vitamine K2 menaquinone, le beta-aminopropionitrile (bAPN) et le Vandetanib (ZD6474 5), ou bien l'application d'ultrasons pulsés de faible intensité. Il est également envisageable d'utiliser une association d'au moins deux de ces modulateurs.

Les quantités efficaces des modulateurs sont à adapter en fonction du résultat recherché et du type et du nombre de modulateurs utilisés ; elles peuvent être comprises en 0.001% et 30% en poids.

Des modulateurs tels que décrits peuvent être utilisés dans les méthodes cosmétiques décrites en association avec des modulateurs des gènes EFEMP1, ECM1, ASPN, HS3ST6, PAPLN, CHSY1 et FLRT2, ou bien des modulateurs des gènes MXRA5, LYZ, CTSL2, PLAU et TIMP1.

Des combinaisons de gènes préférées ont déjà été décrites.

Les modulateurs peuvent être utilisés en association avec d'autres produits, actifs ou excipients. De préférence, ils sont conditionnés sous une forme adaptée à une application topique, par exemple sous forme de pommade, de crème ou d'onguent, ou sous toute forme adaptée au soin telle que lotion, sérum, savon, etc....

Les diverses mises en oeuvre détaillées dans la section relative aux méthodes cosmétiques sont applicables aux utilisations pour les applications cosmétiques décrites ci-dessus.

Les modulateurs décrits ci-dessus peuvent être utilisés notamment dans des applications cosmétiques en vue d'uniformiser le teint, d'homogénéiser la couleur de la peau ou de lutter contre les dyschromies.

Par ailleurs, la présente description décrit des modulateurs du niveau d'expression ou de l'activité du produit d'expression d'au moins un gène dermique choisi parmi les gènes TGFBR2, TGFBI, BMP2, SMAD3, THBS2, TGFBR3, SEMA5A, SMAD7, SOSTDC1, FRAS1, LEPREL1, MATN2, DST, PLOD2, ITGA2, COL6A3, CRTAP, LAMC1, LAMB3, LAMA3, ITGAV et ACTN1 pour une application dans le traitement de taches pigmentaires cutanées, par exemple dans le cadre de traitements thérapeutiques. Lesdites taches peuvent être des taches hyperpigmentaires, ou bien des taches hypopigmentaires. Alternativement, le gène peut être choisi parmi la liste A de gènes dermiques liés à la voie de signalisation TGF-béta - SMAD, ou bien parmi la liste de gènes dermiques B, ou bien parmi les différents sous-groupes de cette dernière liste, à savoir parmi LEPREL1, PLOD2, COL6A3 et CRTAP, ou bien parmi LAMC1, LAMB3 et LAMA3, ou bien parmi FRAS1, MATN2 et DST, ou bien encore parmi ITGA2, ITGAV et ITGB1, ou entre ITGA2 et ITGAV.

Des modulateurs préférés ont déjà été décrits ci-dessus.

Ils peuvent être associés à d'autres produits, actifs ou excipients. De préférence, ils sont conditionnés sous une forme adaptée à une application topique, par exemple sous forme de pommade, de crème ou d'onguent, ou sous toute forme adaptée au soin telle que lotion, sérum, savon, etc....

Dans les différentes méthodes et applications décrites, les taches pigmentaires considérées sont de préférence des taches hyperpigmentaires et tout préférentiellement des lentigos actiniques, solaires ou séniles. Les méthodes et applications décrites sont basées sur la mise en évidence par les inventeurs d'une signature de telles taches pigmentaires.

Cette signature est caractérisée par le fait qu'elle peut être constituée par l'ensemble de la liste ou une partie des gènes cités.

Egalement décrit est l'utilisation de cette signature comme nouveau procédé de sélection et de prédiction du lentigo actinique au travers des déficiences de ses fonctions biologiques. Ce nouveau procédé est basé sur l'étude du niveau d'expression de tout ou partie des gènes décrits dans une lésion hyperpigmentée versus une peau non lésée adjacente.

Cette divulgation s'inscrit également dans le cadre du traitement du lentigo actinique, grâce à l'utilisation de ces gènes comme signature moléculaire des différences d'expression génique existant entre une lésion et la peau saine adjacente. Cette signature constitue également un avantage évident pour déterminer le choix du traitement approprié et mesurer l'effet d'un produit (actif, molécule, extrait naturel), mais aussi d'un procédé (lumière, injection, voie orale) qui soit bénéfique sur la peau. La présente invention permet en effet l'évaluation de l'efficacité d'un produit ou procédé destiné à traiter le lentigo actinique en modulant au niveau de la lésion l'expression de tout ou partie des gènes décrits de sorte à ce que leur profil d'expression se rapproche de celui de la peau saine.

La description consiste aussi en une méthode de criblage de facteurs d'inhibition ou de prévention des lentigos actiniques. Elle consiste à évaluer des composés sur leur pouvoir d'inhibition ou d'augmentation de l'expression des gènes cités et/ou de l'expression ou l'activité des produits protéiques desdits gènes et à sélectionner en tant que facteurs permettant de prévenir, ou traiter le lentigo actinique. La vérification de l'efficacité des composés peut être réalisés sur des modèles cellulaires mono ou co cultures, ou des modèles tri dimensionnels de peau reconstruite, ou de la peau ex vivo, ou in vivo.

La divulgation a aussi pour objet l'utilisation de composés modulant l'expression des gènes identifiés parmi les biomarqueurs du lentigo actinique, pour prévenir ou corriger la lésion afin de retrouver un état normal de la peau, c'est-à-dire retrouver une expression se rapprochant de l'expression de la peau saine non lésée. En particulier, il n'a jamais été proposé des composés agissant sur les protéines identifiées pour prévenir ou traiter des dyschromies pigmentaires (hyperpigmentation ou hypopigmentation). De tels composés existent et sont reportés dans le tableau 3 de l'exemple 3.

Les agents choisis sont notamment des modulateurs négatifs des protéines surexprimées en rapport avec la matrice extracellulaire, notamment des protéines de la voie de signalisation TGF-β - SMAD ou des modulateurs positifs des protéines sous-exprimées.

Parmi les modulateurs négatifs des protéines liées à la matrice extracellulaire et notamment à la voie de signalisation TGF-β - SMAD on pourra citer notamment des inhibiteurs de synthèse, et/ou de la sécrétion et/ou activateur de la dégradation des protéines retrouvés surexprimés dans le lentigo.

A l'inverse parmi les modulateurs positifs, on pourra citer des stimulants de synthèse, des inducteurs de sécrétion ou des inhibiteurs de la dégradation des protéines sous exprimés dans le lentigo.

### Légende des figures :

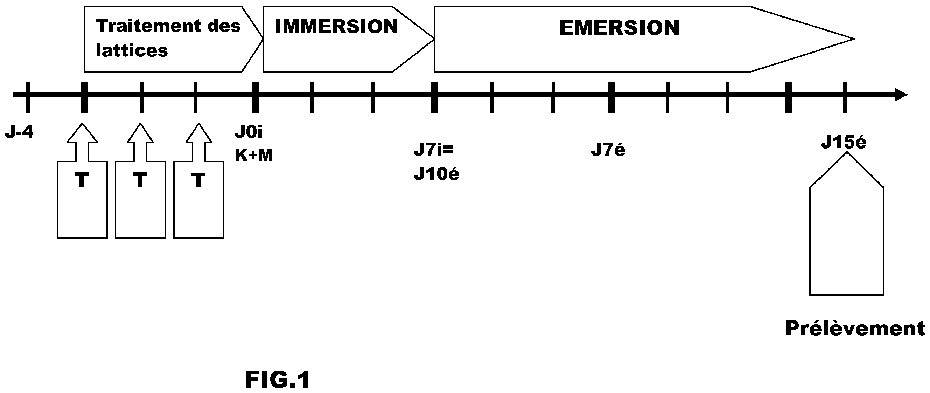

**Figure 1** **:** Cette figure illustre le protocole de traitement des Peaux Reconstruites Pigmentées (PRP) par le TGFbeta. Sur le graphique, T signifie 'traitement au TGFbeta', à 200 pg/ml, avec pour véhicule 4mM de HCl et 1 mg/mL de BSA. Le milieu est du MEM 3F' avec 2% de FBS. Les dermes équivalents sont traités durant 3 jours consécutifs. A J0i, le derme équivalent est ensemencé avec des kératinocytes et des mélanocytes (K+M sur le graphique) et maintenu à immersion durant 7 jours. La culture est ensuite maintenue à émersion, durant 2 semaines.

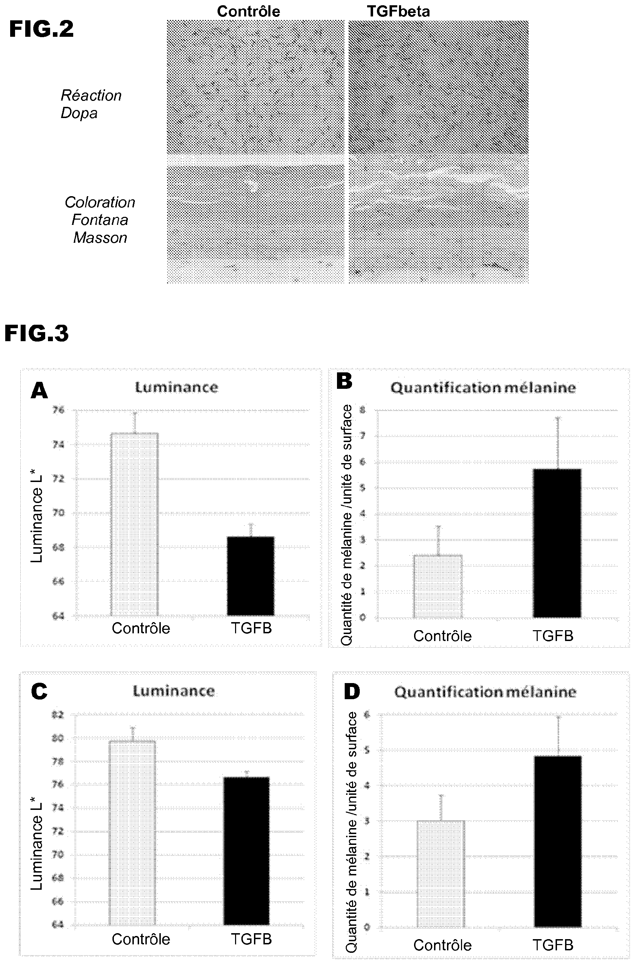

**Figure 2** **:** Cette figure représente des photos des peaux reconstruites pigmentées après traitement avec un contrôle (2% SVF HCI/BSA) ou avec TGFbeta (200 pg/ml) selon le protocole décrit dans l'exemple 4. Les deux photos supérieures correspondent à la réaction Dopa, sur épiderme détaché. Les deux photos inférieures correspondent à une coloration Fontana Masson.
**Figure 3** **:** Cette figure illustre les résultats de luminance (graphes A et C) et de quantification de mélanine (graphes B et D), pour des peaux reconstruites pigmentées traitées conformément au protocole décrit dans l'exemple 4. Le contrôle correspond à un traitement avec 2% de SVF HCI/BSA, TGFB correspond à un traitement avec TGF-beta à 200pg/mL. Les 2 graphes supérieurs et les 2 graphes inférieurs correspondent à deux expériences identiques et indépendantes.

### Partie expérimentale :

### Exemple 1 : Etude transcriptionnelle

Une étude comparative du profil d'expression génique de la peau issue d'une lésion de lentigo actinique (PL) et de peau adjacente non lésée (PN) a été réalisée.

Le but de cette étude est d'identifier des marqueurs pertinents, reproductibles et significatifs reflétant les altérations associées à la formation du lentigo actinique, afin de les utiliser comme cibles pour des traitements efficaces, ou comme biomarqueurs pour analyser l'efficacité d'un traitement donné.

Brièvement, 15 volontaires féminins sains ont été recrutés afin de participer à une étude transcriptomique « full génome » (puces Affymétrix). Pour chaque volontaire, une lésion de type lentigo actinique a été diagnostiquée sur le dos de la main et le diagnostic de lentigo actinique a été vérifié par épiluminescence. Cet examen permet
1°) de vérifier le diagnostic clinique de la lésion (lentigo actinique exclusivement) selon les critères de pattern de la jonction dermo-épidermique (structure pigmentée en réseau, « fingerprint-like structure ») à différencier des éphélides (absence de structure pigmentée en réseau, pigmentation homogène et zones de « moth-eaten edges ») et des kératoses séborrhéiques planes (multiple milia-like cysts or pseudocysts, zones de « moth-eaten edges », pseudofollicular openings et fingerprint-like pattern) [*Menzies et al ; Stolz et al ; Carli et al*]*.*
2°) de délimiter des zones homogènes en terme de structure/pattern à l'intérieur de ces lésions où seront faites les biopsies cutanées,
3°) d'établir un score phénotypique basé sur une analyse d'image quantitative avec le logiciel spécifique qui a été développé sur Matlab^{®} (logiciel SQA, CMLA, ENS Cachan, UMR CNRS 8536).

Une biopsie de 3mm centrée sur la lésion a été réalisée ainsi qu'une biopsie de taille identique sur une zone de peau adjacente non lésée. Les ARN totaux de ces prélèvements ont été extraits et amplifiés. Des sondes ont été générées pour hybridation sur les puces Affymetrix. Des profils d'expression de gènes ont été générés pour chacune des 30 biopsies (2 biopsies par volontaires), et une analyse comparative a été réalisée entre PN et PL par volontaire et sur l'ensemble des volontaires. Les gènes statistiquement exprimés de façon différentielle (moyenne géométrique des patients) ont été compilés dans des listes et regroupés par familles fonctionnelles.

De façon surprenante et inattendue, les inventeurs ont trouvé une expression différentielle pour plusieurs centaines de gènes entre la PN et la PL. Une liste de 437 gènes a été constituée et représente une signature moléculaire large de la lésion lentigo actinique. Parmi les 437 gènes identifiés, 169 gènes se sont révélés régulés de façon positive (« upregulés ») dans la lésion 'lentigo actinique' comparée à la peau saine alors que 269 gènes étaient régulés de façon négative dans le lentigo actinique (« down régulés »).

L'ensemble de ces gènes a été subdivisé en plusieurs familles fonctionnelles.

Parmi les familles fonctionnelles ou les processus biologiques identifiés, les inventeurs ont de façon surprenante trouvé une famille de gènes reflétant au niveau moléculaire un disfonctionnement de la matrice extracellulaire et de la jonction dermo-épidermique dans le lentigo actinique. Ces gènes, regroupés dans cette famille fonctionnelle nommée « Matrice extracellulaire » n'ont jamais été décrits auparavant comme associés au lentigo actinique et sont listés ci-dessous et dans les tableaux 1 et 2 :
Gènes sur-exprimés dans le lentigo actinique par rapport à la peau saine TGFBR2, TGFBI, BMP2, SMAD3, THBS2, TGFBR3, SEMA5A, SMAD7, EFEMP1, ASPN, PAPLN, CHSY1, MXRA5, LYZ, PLAU, TIMP1, FRAS1, LEPREL1, MATN2, DST, ITGA2, COL6A3, CRTAP, LAMC1, LAMB3, LAMA3, ITGAV, ITGB1 et ACTN1.
Gènes sous-exprimés dans le lentigo actinique par rapport à la peau saine SOSTDC1, ECM1, HS3ST6, FLRT2, CTSL2 et PLOD2.

Ces gènes codent pour des composants du derme ou des protéines impliquées dans la synthèse des composants de la matrice extracellulaire, liés au renouvellement ou au remodelage de cette matrice conjonctive, ainsi que des gènes codant pour des protéines matricielles associées à la jonction dermo-épidermique et la zone de la membrane basale. Cette famille contient aussi des gènes liés à la voie du TGFbeta, impliquée dans la synthèse des composants de la matrice extracellulaire. Les gènes de cette famille sont majoritairement sur exprimés dans le lentigo actinique. Cette famille de gènes est tout à fait originale dans un désordre pigmentaire et sous entend un rôle prépondérant du stroma dans le lentigo actinique.

**Tableau 1 : liste des gènes de la famille « matrice extracellulaire » retrouvés surexprimés dans le lentigo actinique**

| Sous famille Voie / fonction | Dénomination | Numéro accession | Nom complet | Taux de modulation |
|---|---|---|---|---|
| | THBS2 | NM_003247 | thrombospondin 2 | **3,41** |
| | TGFBI | NM_000358 | transforming growth factor, beta-induced, 68kDa | **2,22** |
| | BMP2 | NM_001200 | bone morphogenetic protein 2 | **2,03** |
| | SMAD3 | NM_005902 | SMAD family member 3 | **1,76** |
| TGFb/SMAD | TGFBR3 | NM_003243 | Transforming growth factor, beta receptor III | **1,68** |
| | | D50683 | transforming growth factor, beta receptor II (70/80kDa) | **1,61** |
| | TGFBR2 | NM_001024847 | | |
| | | NM_003242 | | |
| | SEMA5A | NM_003966 | sema domain, seven thrombospondin repeats (sémaphorine) (semaphorin 5A, semaphorin F) | **1,56** |
| | SMAD7 | NM_005904 | SMAD family member 7 | **1,52** |
| Collagènes | LEPREL1 | NM_018192 | leprecan-like 1 | **2,64** |
| | COL6A3 | NM_004369 | collagen, type VI, alpha 3 | **1,90** |
| | CRTAP | NM_006371 | cartilage associated protein (LEPREL3) | **1,60** |
| Laminines | LAMC1 | NM_002293 | laminin, gamma 1 (anciennement LAMB2) | **1,96** |
| Laminines | | L25541 | | |
| | LAMB3 | NM_000228 | laminin, beta 3 | **1,94** |
| | | NM_001017402 | | |
| | | NM_001127641 | | |
| | LAMA3 | NM_000227 | laminin, alpha 3 | **1,64** |
| Intégrines | ITGA2 | NM_002203 | integrin, alpha 2 (CD49B, alpha 2 subunit of VLA-2 receptor) | **1,62** |
| | ITGAV | NM_001145000 | integrin, alpha V (vitronectin receptor,) | |
| | | NM_001144999 | | **1,62** |
| | | NM_002210 | | |
| | ITGB1 | NM_133376 | integrin, beta 1 | **1,55** |
| Remodelage matriciel | MXRA5 | NM_015419 | matrix-remodelling associated 5 | **2,40** |
| | LYZ | NM_000239 | lysozyme (renal amyloidosis) | **2,33** |
| | TIMP1 | NM_003254 | TIMP metallopeptidase inhibitor 1 | **2,26** |
| | PLAU | NM_002658 | plasminogen activator, urokinase | **1,81** |
| Protéoglycanes Et glycoprotéine extracellulaire | EFEMP1 | NM_001039348 | EGF-containing fibulin-like extracellular matrix protein 1 (FIBULINE 3) | |
| | | NM_001039349 | | **3,86** |
| | | NM_004105 | | |
| | ASPN | NM_017680 | Asporin | **2,77** |
| | PAPLN | NM_173462 | papilin, proteoglycan-like sulfated glycoprotein | **2,51** |
| | CHSY1 | NM_014918 | carbohydrate (chondroitin) synthase 1 | **1,50** |
| Membrane basale | FRAS1 | NM_025074 | Fraser syndrome 1 | **4,59** |
| | MATN2 | NM_002380 | matrilin 2 | **2,61** |
| | DST | NM_001723 | Dystonin = BPAG1 | **2,24** |
| | | NM_015548 | | |
| | | NM_001130005 | | |
| Actine | ACTN1 | NM_001130004 | actinin, alpha 1 | **1,58** |
| | | NM_001102 | | |

**Tableau 2 : liste des gènes de la famille « matrice extracellulaire » retrouvés sous-exprimés dans le lentigo actinique**

| Sous famille Voie / fonction | Dénomination | Numéro d'accession | Nom complet | Taux de modulation |
|---|---|---|---|---|
| Protéoglycanes | HS3ST6 | NM_001009606 | heparan sulfate (glucosamine) 3-O-sulfotransferase 6 | **0,44** |
| Collagènes | PLOD2 | NM_000935 | procollagen-lysine, 2-oxoglutarate 5-dioxygenase 2 | **0,45** |
| | | NM_182943 | | |
| Protéoglycanes | FLRT2 | NM_013231 | fibronectin leucine rich transmembrane protein 2 | **0,47** |
| Remodelage matriciel | CTSL2 | NM_001333 | cathepsin L2 (*peptidase MEC Degradation*) | **0,54** |
| Protéoglycanes | ECM1 | U65932 | extracellular matrix protein 1 | **0,54** |
| | | NM_004425 | | |
| | | NM_022664 | | |
| TGFb/SMAD | SOSTDC1 | NM_015464 | sclerostin domain containing 1 (ectodin, *BMP antagonist*) | **0,62** |

### Exemple 2 : matériel et méthodes

15 volontaires, femmes, phototypes II à IV, âgées de 50 à 70 ans ont été sélectionnées.

Les lentigos actiniques sont choisis sur le dos de la main, de taille minimum 3mm. Ils sont caractérisés par Epiluminescence. Les différents avantages de la caractérisation par épiluminsecence sont présentés dans l'exemple 1.

Deux biopsies de 3mm de diamètres sont réalisées sur une des mains de chaque patient. Pour chaque volontaire, une des biopsies correspond à la lésion de lentigo actinique (PL) et l'autre à une zone adjacente de peau non lésionnelle (PN) (vérifiée également en épiluminescence).

Les biopsies de 3mm sont placées, dès le prélèvement, dans du RNA later (Qiagen référence 76106) de 16 à 24h à 4°C. Le lendemain les échantillons sont placés à -20°C jusqu'aux étapes d'homogénéisation et d'extraction. A la décongélation, les échantillons sont découpés au scalpel pour faciliter l'homogénéisation, avant le transfert dans le tampon de lyse. L'homogénéisation est réalisée avec un potter (Fisher Labosi ref A6391000) avec des pistons en polypropylène RNase free (Fisher Labosi ref A1419753) permettant l'homogénéisation directe dans les tubes Eppendorf 1,5ml.

L'ARN a été extrait avec les kits RNeasy micro (Qiagen ref : 74004) en suivant les instructions du fournisseur. La quantification des ARNs est réalisée par un dosage ribogreen (Molecular Probes réf R11490). La qualité est validée avec un bio analyseur Agilent 2100 qui donne le ratio des intensités des ARNs ribosomaux 28S sur 18S ainsi qu'un RNA Integrity Number (RIN) qui tient compte de la dégradation des ARNs. Un ARN de bonne qualité a un ratio >1,5 et un RIN > 7.

Une réaction de reverse transcriptase (RT) est réalisée pour obtenir les ADNc correspondants.

Deux sondes par échantillons ont été synthétisées à partir de 50 ng d'ARNs avec une étape d'amplification. Les ADNc sont marqués par fluorochromes et hybridés sur puces à ADN Affymétrix® permettant de révéler le niveau d'expression de l'ensemble des gènes du génome humain. (bio-puces à ADN de type Affymetrix U133A 2.0 U133A 2.0 contenant 54 000 sondes, permettant ainsi l'étude de l'expression de 47000 transcripts, incluant 38500 gènes caractérisés). Affymetrix Microarray suite (Mas 5.0) permet d'obtenir pour chaque transcrit, un signal de détection. Après révélation des hybridations spécifiques et traitement des données brutes (extraction, soustraction du bruit de fond, normalisation), l'expression des gènes est comparée entre la peau saine et la peau lésée. 2 puces Affymetrix HG_U133 Plus 2 ont été hybridées par échantillons.

La qualité de l'hybridation a été effectuée par la méthode AffyPLM (Bolstad *et coll.,* 2005) et par la méthode ACP (Analyses en Composantes Principales).

Les patients ne sont retenus pour la suite de l'analyse que si les 2 puces Affymetrix ont une qualité d'hybridation correcte. Pour la présente étude, 13 patients sur les 15 initiaux ont pu être analysés.

La réalisation d'un profil transcriptomique de la peau saine et de la peau correspondant au lentigo actinique a permis de générer des listes de gènes différentiellement exprimés dans les deux situations, et d'identifier des biomarqueurs du lentigo actinique. Les listes sont générées sous forme de ratio d'expression entre PL (peau lésée) versus PN (peau normale). Le ratio représentant la moyenne géométrique des 13 patients est retenu.

Génération des listes de gènes différentiellement exprimées entre peau lésée et peau saine Etapes :
- Filtre des identifiants Affymetrix (ProbeSets) : seuls les ProbeSets qui sont présents dans les deux réplicats d'au moins une biopsie sont retenus. Après ce filtre 23 968 ProbeSets sont retenus.
- Suppression de l'effet patient : Afin de supprimer l'effet patient observé dans les résultats des analyses différentielles, l'expression de chaque ProbeSet a été divisée par la moyenne géométrique des 4 valeurs des ProbeSets correspondant aux 4 puces.
- Analyse différentielle : elle a été générée en regroupant les listes obtenues par 2 méthodes, l'analyse cNMF (consensus Non Negative Matrix Factorisation(Lee et Seung (1999), Brunet *et coll.* (2004), Fogel *et coll.* (2007), Fogel *et coll.* (2008)).) qui a identifié **2638 ProbeSets** (dont 1521 induits et 1117 réprimés) et la méthode PLS (Partial Least Squares Regression) qui a identifier **610** ProbeSets modulés. L'union des 2 listes a permis d'obtenir une liste de **3248 ProbeSets.**
- Filtre sur la modulation : pour les gènes induits, sélection des ProbeSets ayant une moyenne géométrique des 13 folds corrigés (FC) ≥ 1.5: liste de **562 ProbeSets induits.** Pour les gènes réprimés, sélection des ProbeSets ayant une moyenne géométrique des 13 folds corrigés FC ≤ 0.67 : liste de **807 ProbeSets réprimés.** Au total : 562 + 807 = 1369 ProbeSets modulés, soient **1007 ProbeSets** après élimination des doublons (1002 approche cNMF + 5 approche PLS)
- Filtre sur les 13 patients :visualisation des modulations des 1007 ProbeSets sous forme d'histogrammes et sélection des ProbeSets modulés dans le même sens chez les 13 patients. Liste finale de **132 ProbeSets** qui différencient les biopsies lésionnelles des biopsies non lésionnelles et qui sont modulés dans le même sens chez les 13 patients de l'étude.
- Analyse de la liste des 1007 Probe sets
   ∘ Filtre sur la P value (≤ 0.00001) Afin de ne retenir que les gènes les plus discriminants, nous avons appliqué à la liste des 1002 ProbeSets un filtre sur la P-value, P ≤ 0.00001. Après ce nouveau filtre 827 ProbeSets auxquels sont ajoutés les 5 ProbeSets issus de l'approche PLS 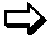 liste de **832 ProbeSets**

Après recherche d'annotations, élimination des gènes non annotés et élimination des doublons, une liste de 437 **gènes** différentiellement exprimés entre PL et PN a été établie. 169 gènes sont sur-exprimés dans le LA et 269 sont sous exprimés.

A l'aide des outils, Gene ontology, PubMed, Scopus les fonctions des gènes sont recherchées et les gènes sont classés par familles fonctionnelles.

### Exemple 3 : modulateurs :

Il existe des composés connus pour moduler dans le sens recherché les protéines dérégulées dans le lentigo. Pour la famille des gènes/protéines de la matrice extracellulaire, on pourra citer :
- des fibrates dont par exemple le fenofibrate qui diminue l'expression de SMAD3,
- des alkaloïdes dont l'oxymatrine qui diminue SMAD3, ou des polyphenols comme par exemple l'acide salvianolique B qui diminue SMAD3 et TGFBR2,
- des extraits naturels notamment des herbes médicinales comme le Wen-pi-tang-hab-wu-ling-san qui diminue SMAD3,
- des composés de la famille des imidazoles antagonistes de TGF-βR1 comme le SB-431542 qui diminue SMAD3,
- certains miRNA, notamment les miR183 et miR29b qui diminuent ITGB1,
- des inhibiteurs de urokinase-type plasminogen activator (uPA) comme le p-aminobenzamidine ou le B428 4- subtituted benzo[b]thiophene-2-carboxamodine qui diminuent l'activité de PLAU,
- des composés de la famille des phenylacetates comme le NaPA qui diminue expression de PLAU,
- des composés de la famille chimique des thiazolidinediones dont par exemple le pioglitazone qui diminue BMP2 et COL6A3,
- des composés de la famille chimique des thiazoles dont par exemple le GW-0742 qui diminue l'expression de BMP2,
- des composés de la famille des tetrazoles comme le Valsartan qui diminue TIMP1.

Concernant la protéine enzymatique PLOD2 de la famille extracellulaire sous exprimée dans le lentigo, on pourra employer les composés suivants pour restaurer son taux :
- des composés de la famille des quinazolines comme le Vandetanib (ZD64745 5) qui augmentent PLOD2.

D'autres types de modulateurs peuvent également être employés pour permettre la correction de l'expression/ quantité ou activité des protéines dérégulées. On peut citer :
- des acide nucléiques (de préférence ARN antisens ou ARNi), anticorps neutralisant,
- des moyens électrique, lumineux, mécanique ou thermique. A titre d'exemple, les ultrasons pulsés à faible intensité (LIPUS) pourront être utilisés pour augmenter la quantité ou l'activité de PLOD2.

A l'inverse, en cas de tache hypopigmentaire, des modulateurs préférés seront ceux augmentant le taux d'expression ou d'activité des protéines issues des gènes TGFBR2, TGFBI, BMP2, SMAD3, THBS2, TGFBR3, SEMA5A, SMAD7, EFEMP1, ASPN, PAPLN, CHSY1, MXRA5, LYZ, PLAU, TIMP1, FRAS1, LEPREL1, MATN2, DST, ITGA2, COL6A3, CRTAP, LAMC1, LAMB3, LAMA3, ITGAV, ITGB1 et ACTN1 et ceux diminuant le taux d'expression ou d'activité des protéines issues des gènes SOSTDC1, ECM1, HS3ST6, FLRT2, CTSL2 et PLOD2.

Des exemples de tels modulateurs sont également répertoriés dans le tableau 3.

**Tableau 3. LISTE DES MODULATEURS DE GENES IMPLIQUE DANS LE LENTIGO ACTINIQUE**

| Sous famille / fonction | Nom | Modulateur gène/protéine | Sens de la modulation |
|---|---|---|---|
| TGFB/ SMAD | TGFBI, transforming growth factor β-induced, 68kDa | Poudre osseuse déminéralisée (Demineralized bone powder, DBP) | Augmente expression |
| TGFB/ SMAD | BMP2, bone morphogenetic protein2 | Pioglitazone | Diminue expr. |
| | | GW0742 | Diminue expr. |
| | | Cristata L flavonoid | augmente expr. |
| TGFB/ SMAD | SMAD3, SMAD family member3 | Fenofibrate | Diminue expression |
| | | Oxymatrine | |
| | | Salvianolic Acid B (Sal B), composant de Danshen (une herbe traditionnelle chinoise utilisée pour les maladies chroniques rénales) antioxydant et protection cellulaire. | |
| | | SB-431542 (un inhibiteur spécifique de TβR-I kinase) | Diminue phosphorylation |
| | | Wen-pi-tang-Hab-Wu-ling-san (WHW) extrait | |
| TGFB/ SMAD | TGFBR2, transforming growth factor, β receptor II (70/80kDa) | Salvianolic acid B (SA-B) | Diminue expression |
| TGFB/ SMAD | SMAD7, SMAD family member 7 | Oxymatrine | augmente expression |
| | | 3-deoxyglucosone (Advanced glycation endproduct precursor) | |
| | | Tetrandrine | |
| | | N-acetyl-seryl-aspartyl-lysyl-proline(Ac-SDKP) | |
| Collagène | COL6A3, collagen, type VI, alpha 3 | Pioglitazone | Diminue expression |
| Intégrine | ITGB1, integrin, beta | miR-183 , miR-29b | Diminue expression |
| Remodelage matriciel | LYZ, lysozyme (renal amyloidosis) | Pituitary adenylate cyclase-activating polypeptide (PACAP) PACAP38 | Augmente expression |
| Remodelage matriciel | TIMP1, metallopeptidase inhibitor 1 | Valsartan , (angiotensin II type 1 receptor blocker ARB) Extrait végétal de Lupinus albus LU10 | Diminue expression |
| | | Poudre osseuse déminéralisée (Demineralized bone powder, DBP) | Augmente express. |
| Remodelage matriciel | PLAU, plasminogen activator, urokinase | Sodium phenylacetate (NaPA) | Diminue expression |
| | | P-aminobenzamidine (Urokinase-type plasminogen activator(uPA) inhibitor):B428 4, B392 | Diminue activité |
| | | -substituted benzo[b]thiophene-2-carboxamidines (Urokinase-type plasminogen activator(uPA) inhibitor | |
| | | Thienopyridine SR 25989 (Angiogenesis inhibitor) esterified derivative of ticlopidine, Notoginsenoside R1 (issu de PANAX notoginseng) | Augmente expression |
| Assimilé Protéoglycane | ASPN, Asporin | letrozole, anastrozole | Augmente expression |
| Membrane basale | MATN2, matrilin 2 | vitamine K2 menaquinone | Augmente expression |
| Collagène | PLOD2 , procollagen-lysine, 2-oxoglutarate 5-dioxygenase 2 | beta-aminopropionitrile (bAPN) | Diminue expression |
| | | Low-intensity pulsed ultrasound (LIPUS) ultrasons pulsés de faible intensité | Augmente expr. /activité |
| | | Vandetanib (ZD6474 5) | |
| Remodelage matriciel | CTSL2 ,cathepsin L2 | Derivés phenylalanine | Diminue activité |

### Exemple 4 : Stimulation des la pigmentation des peaux reconstruites pigmentées par traitement au TGF-beta

### 1) Protocole de traitement.

Le Protocole de traitement des Peaux Reconstruites Pigmentées (PRP) au TGFBETA est illustré à la figure 1. Il est le suivant :
Des dermes équivalents (lattices) contenant des fibroblastes vivants dans une matrice de collagène, sont collés sur le fond d'une boite de pétri (J-4) et sont traités au TGFbeta1 (T sur la figure 1) à la concentration de 200pg/ml (véhicule : 4 mM de HCl et 1 mg/mL de BSA ; milieu : MEM 3F' avec 2% de FBS), pendant 3 jours (J-3 ; J-2 et J-1 sur la figure 1). Puis l'épiderme pigmenté est reconstruit sur le derme équivalent en ensemençant les kératinocytes et les mélanocytes (J0). La culture est maintenue à l'immersion pendant 1 semaine et à l'émersion pendant 2 semaines (Fig. 1).

Les échantillons sont prélevés pour analyse de la pigmentation :
- L'intégration et la morphologie des mélanocytes sont observés après réaction Dopa sur épiderme détaché ;
- La mesure colorimétrique de la de Luminance renseigne sur le degré de clarté des échantillons (plus L*est faible plus les échantillons sont foncés) ;
- La présence de mélanine est observée après coloration Fontana Masson de coupes de peau ; et
- La quantité de mélanine présente dans l'épiderme est mesurée par analyse d'images.

### 2) Résultats :

Les résultats montrent qu'après un traitement au TGFbeta 1 à 200 pg/ml et comparativement au contrôle (Figures 2 et 3):
- les mélanocytes sont toujours présents dans les épidermes et conservent une morphologie correcte dendritique ;
- des dépôts de mélanine sont plus importants dans l'épiderme ;
- La luminance des échantillons diminue (brunissement des peaux reconstruites traitées) ;
- Le contenu en mélanine est augmenté, visiblement par observation des coupes colorées au Fontana Masson et objectivée par quantification de la mélanine par analyse d'images.

L'expérience est renouvelée une seconde fois et confirme les résultats obtenus (Fig. 3).

Les résultats démontrent qu'une activation de la voie de signalisation du TGF-beta est responsable d'une pigmentation plus importante de la peau comparativement à l'absence d'activation de cette voie de signalisation.

### Bibliographie :

Andersen WK, Labadie RR, Bhawan J. « Histopathology of solar lentigines of the face: a quantitative study." J Am Acad Dermatol. 1997 Mar;36(3 Pt 1):444-7.
Ber Rahman S, Bhawan J. Lentigo. Int J Dermatol. 1996 Apr;35(4):229-39. Review.
Berking C, Takemoto R, Schaider H, Showe L, Satyamoorthy K, Robbins P, Herlyn M. "Transforming Growth Factor-β1 Increases Survival of Human Melanoma through Stroma Remodeling". Cancer Res (2001); 61: 8306-8316.
Cario-Andre M, Lepreux S, Pain C, Nizard C, Noblesse E, Taïeb A. « Perilesional vs. lesional skin changes in senile lentigo. » J Cutan Pathol. 2004 Jul;31(6):441-7.
Carli P. Salvini C. "Lentigines including lentigo simplex, reticulated lentigo, and actinic lentigo." In Color Atlas of melanocytic lesions of the skin. Soyer H.P., Argenziano G., Hofman-Wellenhof R. , Johr R. Springer-Verlag Berlin Heidelberg 2007: 290-294.
Kawata Y, Suzuki H, Higaki Y, Denisenko O, Schullery D, Abrass C, Bomsztyk; "Bcn-1 Element-dependent activation of the laminin gamma 1 chain gene by the coopérative action of transcription factor E3 (TFE3) and Smad proteins." J Biol Chem. 2002; 277(13):11375-84.
Menzies SW, Crotty KA, Ingvar C, McCarthy WH. "Benign pigmented macules." In An atlas of surface microscopy of pigmented skin lesions: Demoscopy. Eds Menzies SW, Crotty KA, Ingvar C, McCarthy WH. McGraw-Hill book company Australia Pty Limited, North Ryde, Australia. 2003 : pp 53-60
Montagna W, Hu F, Carlisle K. «A reinvestigation of solar lentigines ». Arch Dermatol. 1980 Oct;116(10):1151-4.
Stolz W, Braun-Falco O, Bilek P, Landthaler M, Burgdorf WHC, Cognetta AB. "Differential diagnosis of pigmented skin lesions" In Color atlas of dermatology . Eds Stolz W, Braun-Falco O, Bilek P, Landthaler M, Burgdorf WHC, Cognetta AB. Blackwell Wissenschafts- Verlag, Berlin, Germany.2002: pp41-66.
Verrecchia F, Chu ML, Mauviel A. "Identification of novel TGF-beta /Smad gene targets in dermal fibroblasts using a combined cDNA microarray/promoter transactivation approach". J. Biol. Chem. (2001) 276 : 17058-17062.

## Revendications

1. Méthode de caractérisation d'une tache pigmentaire cutanée apparente ou soupçonnée chez un être humain, comprenant la comparaison des niveaux d'expression, dans des échantillons de peau issue de ladite tache et de peau adjacente non lésée, au sein des cellules du derme, d'au moins un gène dermique lié à la matrice extracellulaire choisi parmi :
A. la liste constituée des gènes TGFBR2, TGFBI, BMP2, SMAD3, THBS2, TGFBR3, SEMA5A, SMAD7 et SOSTDC1, ou bien parmi
B. la liste constituée des gènes FRAS1, LEPREL1, MATN2, DST, PLOD2, ITGA2, COL6A3, CRTAP, LAMC1, LAMB3, LAMA3, ITGAV, ITGB1 et ACTN1,.
où ladite tache pigmentaire cutanée est un lentigo actinique.

2. Méthode selon la revendication 1, comprenant la comparaison des niveaux d'expression d'au moins deux gènes distincts, de préférence d'au moins cinq gènes distincts choisis parmi l'une et / ou l'autre des listes A et B.

3. Méthode selon la revendication 1, où ledit gène est choisi parmi la liste constituée des gènes TGFBR2, TGFBI, BMP2, SMAD3, THBS2 et TGFBR3.

4. Méthode selon la revendication 1, où ladite tache est confirmée comme lentigo actinique quand le niveau d'expression est
- supérieur dans l'échantillon de peau issue de la tache par rapport au niveau dans l'échantillon de peau adjacente non lésée, si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et
- inférieur dans l'échantillon de peau issue de la tache par rapport au niveau dans l'échantillon de peau adjacente non lésée, si le gène est choisi parmi SOSTDC1 et PLOD2.

5. Méthode in vitro d'évaluation de l'efficacité d'un traitement des lentigos actiniques, comprenant la comparaison des niveaux d'expression au sein des cellules du derme dans un échantillon de peau issue d'un tel lentigo, avant et après traitement, d'au moins un gène dermique lié à la matrice extracellulaire choisi parmi la liste constituée des gènes TGFBR2, TGFBI, BMP2, SMAD3, THBS2, TGFBR3, SEMA5A, SMAD7 et SOSTDC1, ou bien parmi la liste constituée des gènes FRAS1, LEPREL1, MATN2, DST, PLOD2, ITGA2, COL6A3, CRTAP, LAMC1, LAMB3, LAMA3, ITGAV, ITGB1 et ACTN1.

6. Méthode selon la revendication 5, où ledit traitement est considéré comme efficace pour le traitement de lentigos actiniques quand le niveau d'expression est :
- inférieur après traitement par rapport au niveau d'expression avant traitement, si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 et ACTN1, et
- supérieur après traitement par rapport au niveau d'expression avant traitement, si le gène est choisi parmi SOSTDC1 et PLOD2.

7. Méthode *in vitro* d'évaluation de l'efficacité d'un traitement des lentigos actiniques, comprenant la comparaison, avant et après traitement, du niveau d'expression au sein des cellules du derme dans un modèle cellulaire représentatif de la peau, d'au moins un gène dermique lié à la matrice extracellulaire choisi parmi la liste constituée des gènes TGFBR2, TGFBI, BMP2, SMAD3, THBS2, TGFBR3, SEMA5A, SMAD7 et SOSTDC1, ou bien parmi la liste constituée des gènes FRAS1, LEPREL1, MATN2, DST, PLOD2, ITGA2, COL6A3, CRTAP, LAMC1, LAMB3, LAMA3, ITGAV, ITGB1 et ACTN1, ou bien du niveau d'expression ou d'activité du produit d'expression dudit gène choisi.

8. Méthode cosmétique de traitement ou de prévention de lentigo actinique non pathologique de la peau humaine, comprenant la modulation du niveau d'expression ou de l'activité d'un gène dermique lié à la matrice extracellulaire, où ledit gène est choisi parmi la liste constituée des gènes TGFBR2, TGFBI, BMP2, SMAD3, THBS2, TGFBR3, SEMA5A, SMAD7 et SOSTDC1 ou bien parmi la liste constituée des gènes FRAS1, LEPREL1, MATN2, DST, PLOD2, ITGA2, COL6A3, CRTAP, LAMC1, LAMB3, LAMA3, ITGAV et ACTN1.

9. Méthode selon la revendication 8, où ladite méthode comprend la modulation d'au moins deux gènes, de préférence d'au moins cinq gènes distincts, choisis parmi la liste constituée des gènes TGFBR2, TGFBI, BMP2, SMAD3, THBS2, TGFBR3, SEMA5A, SMAD7 et SOSTDC1 et/ou parmi la liste constituée des gènes FRAS1, LEPREL1, MATN2, DST, PLOD2, ITGA2, COL6A3, CRTAP, LAMC1, LAMB3, LAMA3, ITGAV et ACTN1, où ladite modulation est une inhibition si le gène est choisi parmi TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV et ACTN1, et une augmentation du niveau d'expression ou de l'activité si le gène est choisi parmi SOSTDC1 et PLOD2.

10. Utilisation d'un modulateur du niveau d'expression ou de l'activité du produit d'expression d'au moins un gène dermique choisi parmi la liste constituée des gènes TGFBR2, TGFBI, BMP2, SMAD3, THBS2, TGFBR3, SEMA5A, SMAD7 et SOSTDC1, ou bien parmi la liste constituée des gènes FRAS1, LEPREL1, MATN2, DST, PLOD2, ITGA2, COL6A3, CRTAP, LAMC1, LAMB3, LAMA3, ITGAV et ACTN1, pour une application cosmétique dans le traitement de lentigos actiniques non-pathologiques, ledit modulateur étant un antisens, un microARN ou un siRNA dirigé contre le gène dermique choisi et inhibant l'expression dudit gène.

## Patentansprüche

1. Verfahren zur Charakterisierung eines sichtbaren oder vermuteten Hautpigmentfleckens bei einem Menschen, das den Vergleich der Expressionsniveaus in Dermiszellen von wenigstens einem mit der extrazellulären Matrix in Verbindung stehenden Hautgen in Proben aus Haut, die aus diesem Pigmentfleck stammt, und in Proben aus gesunder benachbarter Haut umfasst, das ausgewählt ist aus:
A. der Liste der Gene TGFBR2, TGFBI, BMP2, SMAD3, THBS2, TGFBR3, SEMA5A, SMAD7 und SOSTDC1 oder aus
B. der Liste der Gene FRAS1, LEPREL1, MATN2, DST, PLOD2, ITGA2, COL6A3, CRTAP, LAMC1, LAMB3, LAMA3, ITGAV, ITGB1 und ACTN1,
bei dem der Hautpigmentfleck eine aktinische Lentigo ist.

2. Verfahren nach Anspruch 1, das den Vergleich der Expressionsniveaus von wenigstens zwei verschiedenen Genen, vorzugsweise von wenigstens fünf verschiedenen Genen umfasst, die aus der einen und/oder der andern Liste A und B ausgewählt sind.

3. Verfahren nach Anspruch 1, bei dem das Gen aus der Liste der Gene TGFBR2, TGFBI, BMP2, SMAD3, THBS2 und TGFBR3 ausgewählt ist.

4. Verfahren nach Anspruch 1, bei dem der Pigmentfleck als aktinische Lentigo bestätigt ist, wenn das Expressionsniveau
- in der Hautprobe, die aus dem Pigmentfleck stammt, bezüglich des Niveaus in der Probe aus gesunder benachbarter Haut höher ist, wenn das Gen aus TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 und ACTN1 ausgewählt ist, und
- in der Hautprobe, die aus dem Pigmentfleck stammt, bezüglich des Niveaus in der Probe aus gesunder benachbarter Haut niedriger ist, wenn das Gen aus SOSTDC1 und PLOD2 ausgewählt ist.

5. In-vitro-Verfahren zur Bewertung der Wirksamkeit einer Behandlung von aktinischen Lentigines, das vor und nach der Behandlung den Vergleich der Expressionsniveaus in Dermiszellen von wenigstens einem mit der extrazellulären Matrix in Verbindung stehenden Hautgen in einer aus einer derartigen Lentigo stammenden Hautprobe umfasst, das aus der Liste der Gene TGFBR2, TGFBI, BMP2, SMAD3, THBS2, TGFBR3, SEMA5A, SMAD7 und SOSTDC1 oder aus der Liste der Gene FRAS1, LEPREL1, MATN2, DST, PLOD2, ITGA2, COL6A3, CRTAP, LAMC1, LAMB3, LAMA3, ITGAV, ITGB1 und ACTN1 ausgewählt ist.

6. Verfahren nach Anspruch 5, bei dem die Behandlung als wirksam zur Behandlung von aktinischen Lentigines angesehen wird, wenn das Expressionsniveau
- nach der Behandlung bezüglich des Expressionsniveaus vor der Behandlung niedriger ist, wenn das Gen aus TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 und ACTN1 ausgewählt ist, und
- nach der Behandlung bezüglich des Expressionsniveaus vor der Behandlung höher ist, wenn das Gen aus SOSTDC1 und PLOD2 ausgewählt ist.

7. In-vitro-Verfahren zur Bewertung der Wirksamkeit einer Behandlung von aktinischen Lentigines, das vor und nach der Behandlung den Vergleich des Expressionsniveaus in Dermiszellen in einem für die Haut repräsentativen Zellmodell von wenigstens einem mit der extrazellulären Matrix in Verbindung stehenden Hautgen, das aus der Liste der Gene TGFBR2, TGFBI, BMP2, SMAD3, THBS2, TGFBR3, SEMA5A, SMAD7 und SOSTDC1 oder aus der Liste der Gene FRAS1, LEPREL1, MATN2, DST, PLOD2, ITGA2, COL6A3, CRTAP, LAMC1, LAMB3, LAMA3, ITGAV, ITGB1 und ACTN1 ausgewählt ist, oder den Vergleich des Expressions- oder Aktivitätsniveaus des Expressionsprodukts des ausgewählten Gens umfasst.

8. Kosmetisches Verfahren zur Behandlung oder Vorbeugung von nicht pathologischer aktinischer Lentigo der menschlichen Haut, das die Modulation des Expressions- oder Aktivitätsniveaus eines mit der extrazellulären Matrix in Verbindung stehenden Hautgens umfasst, bei dem das Gen aus der Liste der Gene TGFBR2, TGFBI, BMP2, SMAD3, THBS2, TGFBR3, SEMA5A, SMAD7 und SOSTDC1 oder aus der Liste der Gene FRAS1, LEPREL1, MATN2, DST, PLOD2, ITGA2, COL6A3, CRTAP, LAMC1, LAMB3, LAMA3, ITGAV, ITGB1 und ACTN1 ausgewählt ist.

9. Verfahren nach Anspruch 8, bei dem das Verfahren die Modulation von wenigstens zwei, vorzugsweise von wenigstens fünf verschiedenen Genen umfasst, die aus der Liste der Gene TGFBR2, TGFBI, BMP2, SMAD3, THBS2, TGFBR3, SEMA5A, SMAD7 und SOSTDC1 und/oder aus der Liste der Gene FRAS1, LEPREL1, MATN2, DST, PLOD2, ITGA2, COL6A3, CRTAP, LAMC1, LAMB3, LAMA3, ITGAV, ITGB1 und ACTN1 ausgewählt sind, bei dem die Modulation eine Hemmung ist, wenn das Gen aus TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 und ACTN1 ausgewählt ist, und eine Erhöhung des Expressions- oder Aktivitätsniveaus ist, wenn das Gen aus SOSTDC1 und PLOD2 ausgewählt ist.

10. Verwendung eines Modulators des Expressions- oder Aktivitätsniveaus des Expressionsprodukts von wenigstens einem Hautgen, das aus der Liste der Gene TGFBR2, TGFBI, BMP2, SMAD3, THBS2, TGFBR3, SEMA5A, SMAD7 und SOSTDC1 oder aus der Liste der Gene FRAS1, LEPREL1, MATN2, DST, PLOD2, ITGA2, COL6A3, CRTAP, LAMC1, LAMB3, LAMA3, ITGAV und ACTN1 ausgewählt ist, für eine kosmetische Aufbringung bei der Behandlung von nicht pathologischen aktinischen Lentigines, wobei der Modulator eine gegen das ausgewählte Hautgen gerichtete und die Expression dieses Gens hemmende Antisense-RNA, microRNA oder siRNA ist.

## Claims

1. A method for characterizing a known or suspected cutaneous pigmentary spot in a human being, comprising comparing levels of expression, in skin samples obtained from said spot and from adjacent undamaged skin, within cells of the dermis, of at least one dermal gene linked to the extracellular matrix selected from:
A. the list constituted by the genes TGFBR2, TGFBI, BMP2, SMAD3, THBS2, TGFBR3, SEMA5A, SMAD7 and SOSTDC1; or from
B. the list constituted by the genes FRAS1, LEPREL1, MATN2, DST, PLOD2, ITGA2, COL6A3, CRTAP, LAMC1, LAMB3, LAMA3, ITGAV, ITGB1 and ACTN1,
wherein said cutaneous pigmentary spot is actinic lentigo.

2. The method according to claim 1, comprising comparing the levels of expression of at least two distinct genes, preferably of at least five distinct genes selected from one and/or the other of lists A and B.

3. The method according to claim 1, wherein said gene is selected from the list constituted by the genes TGFBR2, TGFBI, BMP2, SMAD3, THBS2 and TGFBR3.

4. The method according to claim 1, wherein said spot is confirmed as a actinic lentigo when the level of expression is:
- higher in the skin sample obtained from the spot compared with the level in the sample of adjacent undamaged skin, if the gene is selected from TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 and ACTN1, and
- lower in the skin sample obtained from the spot compared with the level in the sample of adjacent undamaged skin, if the gene is selected from SOSTDC1 and PLOD2.

5. An *in vitro* method for evaluating the efficacy of a treatment for actinic lentigo, comprising comparing the levels of expression within cells of the dermis in a skin sample obtained from said lentigo, before and after treatment, of at least one dermal gene linked to the extracellular matrix selected from the list constituted by the genes TGFBR2, TGFBI, BMP2, SMAD3, THBS2, TGFBR3, SEMA5A, SMAD7 and SOSTDC1, or from the list constituted by the genes FRAS1, LEPREL1, MATN2, DST, PLOD2, ITGA2, COL6A3, CRTAP, LAMC1, LAMB3, LAMA3, ITGAV, ITGB1 and ACTN1.

6. The method according to claim 5, wherein said treatment is considered to be effective for the treatment of actinic lentigines when the level of expression is:
- lower after treatment compared with the level of expression before treatment, if the gene is selected from TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV, ITGB1 and ACTN1, and
- higher after treatment compared with the level of expression before treatment, if the gene is selected from SOSTDC1 and PLOD2.

7. An *in vitro* method for evaluating the efficacy of a treatment of actinic lentigines, comprising comparing, before and after treatment, the level of expression within cells of the dermis, in a cellular model representing the skin, of at least one dermal gene linked to the extracellular matrix selected from the list constituted by the genes TGFBR2, TGFBI, BMP2, SMAD3, THBS2, TGFBR3, SEMA5A, SMAD7 and SOSTDC1, or from the list constituted by the genes FRAS1, LEPREL1, MATN2, DST, PLOD2, ITGA2, COL6A3, CRTAP, LAMC1, LAMB3, LAMA3, ITGAV, ITGB1 and ACTN1, or the level of expression or activity of an expression product of said selected gene.

8. A cosmetic method for the treatment or prevention of a non-pathological actinic lentigo of human skin, comprising modulating the level of expression or activity of a dermal gene linked to the extracellular matrix, where said gene is selected from the list constituted by the genes TGFBR2, TGFBI, BMP2, SMAD3, THBS2, TGFBR3, SEMA5A, SMAD7 and SOSTDC1 or from the list constituted by the genes FRAS1, LEPREL1, MATN2, DST, PLOD2, ITGA2, COL6A3, CRTAP, LAMC1, LAMB3, LAMA3, ITGAV and ACTN1.

9. The method according to claim 8, wherein said method comprises the modulation of at least two genes, preferably of at least five distinct genes, selected from the list constituted by the genes TGFBR2, TGFBI, BMP2, SMAD3, THBS2, TGFBR3, SEMA5A, SMAD7 and SOSTDC1 and/orfrom the list constituted by the genes FRAS1, LEPREL1, MATN2, DST, PLOD2, ITGA2, COL6A3, CRTAP, LAMC1, LAMB3, LAMA3, ITGAV and ACTN1, wherein said modulation is an inhibition if the gene is selected from TGFBR2, TGFBI, BMP2, SMAD3, THBS2, TGFBR3, SEMA5A, SMAD7, FRAS1, LEPREL1, MATN2, DST, ITGA2, COL6A3, CRTAP, LAMC1, LAMB3, LAMA3, ITGAV and ACTN1, and an increase in the level of expression or activity if the gene is selected from SOSTDC1 and PLOD2.

10. Use of a modulator of the level of expression or activity of an expression product of at least one dermal gene selected from the list constituted by the genes TGFBR2, TGFBI, BMP2, SMAD3, THBS2, TGFBR3, SEMA5A, SMAD7 and SOSTDC1, or from the list constituted by the genes FRAS1, LEPREL1, MATN2, DST, PLOD2, ITGA2, COL6A3, CRTAP, LAMC1, LAMB3, LAMA3, ITGAV and ACTN1, for a cosmetic application in the treatment of non-pathological actinic lentigines, wherein said modulator is an antisense, a microRNA or a siRNA targeting the selected dermal gene and inhibiting the expression of said gene.
